# EUROPEAN PATENT SPECIFICATION

(11) **EP 0 550 491 B1**
(45) Date of publication and mention of the grant of the patent: **15.03.1995**
(21) Application number: 91916170.3
(22) Date of filing: 25.09.1991
(51) Int. Cl.: C12Q 1/68

(54) **TEST TO DETERMINE AN ORGANISM'S SPECIES AND/OR POPULATION IDENTITY BY DIRECT NUCLEOTIDE SEQUENCE ANALYSIS OF DEFINED SEGMENTS OF ITS GENOME**
TEST ZUR BESTIMMUNG DER SPEZIES UND/ODER DER BESTANDSIDENTITÄT EINES ORGANISMUS MITTELS DER SEQUENZIERUNG VON BESTIMMTEN SEGMENTEN SEINES GENOMS
TEST POUR DETERMINER LES ESPECES D'ORGANISME ET/OU L'IDENTITE D'UNE POPULATION GRACE A UNE ANALYSE DIRECTE DE LA SEQUENCE DE NUCLEOTIDES DE CERTAINS SEGMENTS PARTICULIERS DU GENOME

(30) Priority: 26.09.1990 CA 2026264
(43) Date of publication of application: 14.07.1993
(73) Proprietor: BIO-ID CORPORATION, St. John's, Newfoundland A1C 557 (CA)
(72) Inventor: Davidson, William Scott, St. John's, Newfoundland A1B 2W1 (CA); Bartlett, Sylvia Ernestine, Mount Pearl, Newfoundland A1N 2M6 (CA)
(74) Representative: Orès, Bernard
(86) International application number: CA9100345
(87) International publication number: WO9205277

(56) References cited:
- EP-A- 120 658
- EP-A- 335 633
- JOURNAL OF GENERAL MICROBIOLOGY, vol. 136, no. 9, 18 September 1990, Reading (GB); T. ROGALL et al., pp. 1915-1920#
- JOURNAL OF CLINICAL MICROBIOLOGY, vol. 28, no. 9, September 1990, Washington, DC (US); K.H. WILSON et al., pp. 1942-1946#
- PROCEEDINGS OF THE NATL. ACADEMY OD SCIENCES USA, vol. 86, August 1989, Washington, DC (US); T.D. KOCHER et al., pp. 6196-6200#
- AMERICAN ZOOLOGIST, vol. 28, no. 4, December 1988, Charlottesville, VA (US); A. MEYER et al., p. 35A#
- CANADIAN JOURNAL OF FISHERIES & AQUATIC SCIENCES, vol. 48, no. 2, 1991, Ottawa, ON (CA); S.E. BARTLETT et al., pp. 309-317#

## Description

### (i) Technical Field To Which The Invention Relates

This invention relates to means and methods for the determination of the species of an organism (sample) and/or its population identity by direct nucleotide sequence analysis of defined segments of its genome.

### (ii) Background Art

Many government agencies require a means of identifying the species origin of meat or other biological specimens. For example, law enforcement agencies need to know if a blood stain comes from a human or an animal. Food inspectors check meat for accurate labelling for economic (e.g. selling cheaper meats, e.g. horse for beef) and religious (e.g. pork for beef) reasons. Wildlife officers must be able to identify the species type of meat to detect animals that have been obtained illegally (i.e. hunted without a permit, out of season, or the quota exceeded). In addition, customs officers require methods for identifying restricted, endangered species or products derived from them. There is therefore, a need for a rapid, accurate and reliable method for determining the species origin of a biological sample.

Currently there are three standard procedures for identifying the animal origin of meat, but each has major drawbacks. The most commonly recommended method involves protein electrophoresis. The soluble proteins are detected by general protein or specific enzyme activity stains after electrophoresis using starch gels, polyacrylamide gels or cellulose acetate strips. This method only works with fresh or frozen tissue. Immunological procedures rely on antibodies raised against soluble proteins. Simple Ouchterlony analyses usually suffer from cross-reaction of proteins from related organisms. This procedure may indicate the family of organisms to which the specimen belongs, but rarely can it identify the species. Enzyme-linked immunosorbent assays have been developed that can detect pork or poultry in cooked and canned meat products. However, the polyclonal antibodies raised against the chicken heat-resistant antigens could not discriminate between turkey and chicken products. The third method depends on the ability to separate warer-soluble muscle proteins by high-performance liquid chromatography. This procedure has the advantage of being able to estimate the relative proportions of raw meats in mixtures, but it is only applicable to fresh or freshly frozen meats.

The disadvantages of each of the methods outlined above are a consequence of their dependence on the ability to characterise proteins. Many proteins are heat-labile, lose their biological activity soon after death, are subject to modification in different cell types, and their presence is a function of the cell type being examined. It would be preferable to analyze DNA rather than proteins for identifying the species origin of an unknown sample. DNA carries an organism's genetic information. It is the same in all cell types of an animal. Thus, it does not matter if one isolates DNA from a blood stain, a muscle extract, or a liver sample. The information content of DNA is also greater than that of proteins. Information is lost as one goes from DNA to protein because of the degeneracy of the genetic code. Moreover, electrophoretic, immunological, and chromatographic methods do not detect all the amino acid differences that may occur between different proteins (e.g. electrophoresis will only detect changes in the net charge of two proteins and immunological techniques require different antigenic determinants to be present on the proteins under investigation). DNA is a remarkably stable molecule. It has been isolated from animal skins from the nineteenth century, mummified tissue and a 7,000 year old human brain.

The keys to identifying different species normally rely heavily on morphological characteristics. However, when an animal has been killed for food or sport, these species-specific markers are often destroyed or removed from the animal. This presents problems for government agencies (e.g., Canadian Department of Fisheries and Oceans, Canadian Wildlife Service, Health and Welfare Canada, and other international bodies) who are involved in determining the species type of the animal or meat to enforce species conservation or health-related regulations. There is thus a great need for a reliable and accurate method for determining the identity of the species of an organism when it is not possible to do so using the normal morphological markers.

Four commercially important tuna species in the genus *Thunnus* are caught commercially off the east coast of Canada. The harvest of bluefin tuna (*T*.*thunnus*) is regulated, but of bigeye (*T*.*obesus*), yellowfin (*T*.*albacares*), or albacore (*T*.*alalunga*) is not. Enforcement of the regulations governing the bluefin fishery has been difficult because of the close genetic relationships among these species and the ease with which morphological characters may be removed once a fish has been landed. Isoelectric focusing of water-soluble muscle proteins does not resolve these four tuna species beyond two groups: one consisting of bluefin and yellowfin and the other comprising bigeye and albacore.

The bluefin tuna (*Thunnus thynnus*) is prized as one of the world's best marine sports fish and commands top prices in the lucrative Japanese *sashimi* market. The largest member of the family Scombridae, bluefin tuna is one of four species of *Thunnus* that are caught commercially off Canada's east coast. The other tuna species found in this region are big-eye (*T*.*obesus*), yellowfin (*T*.*albacares*), and albacore (*T*.*alalunga*). A fear that too many juvenile bluefin tuna were being caught brought about the formation of the International Commission for the Conservation of Atlantic Tunas (ICCAT) in 1969 and regulations governing the minimum size and limiting the total allowable catch of bluefin were introduced in the mid 1970's. The Canadian Department of Fisheries and Oceans has taken additional measures to maintain the bluefin tuna fishery within Canadian waters and these include restricting the number of licences issued, controlling the type of gear used, and limiting the number of bluefin tuna that may be taken per boat per day.

With the value of an individual bluefin tuna ranging from $1,000 to $20,000 (1989 Canadian dollars) depending on the size and condition of the fish, it is not surprising that a black market in illegally caught bluefin tuna has arisen. This has made it difficult to maintain the quotas required to conserve and expand the bluefin tuna fishery. Enforcement has also been a problem because it is very difficult to tell a bluefin from a big-eye, yellowfin, or albacore (especially small fish), as all of these species are quite similar in appearance. Moreover, the usual characters used to differentiate between these species are skeletal and visceral in nature (e.g. gillraker counts) and these identifying features are removed once the fish has been landed and gutted.

It is far preferable to examine DNA, the molecule of heredity, rather than proteins. DNA is the same in all cell types of an organism whereas proteins vary from tissue to tissue. DNA is remarkably stable and the amount of information available for analysis decreases as one goes from DNA to protein. This is because of the degeneracy of the genetic code and because protein electrophoresis only detects mutations that change the overall charge of the molecule.

Some solutions to the problem of establishing the genetic identify of a strain of an animal have been proposed. Some of the tests employed for such determinations rely upon the identification of polymorphic proteins in the plasma, from the surface of cells, or extracted from within the cells of the species in question. However, most of the tests are based on the analysis of a protein or its activity, and so it is the gene product and not the gene itself which is the subject of the investigation. It would therefore be preferable to analyze the gene directly rather than the product of its expression because of the degeneracy that is inherent in the process by which genetic information is expressed.

The flow of genetic information in cells is well known. The information directing the biosynthesis of proteins is encoded in the nucleotide sequences of DNA known as genes. The DNA of the cell may be viewed as the storage form of the genetic information. The DNA molecules are large, chemically-stable, easily replicated and contain many gene sequences.

A problem exists in the isolation and purification of the specific gene or nucleotide sequence containing the genetically coded information for the amino acid sequence of the desired protein. DNA exists in all living cells in the form of extremely high molecular weight chains of nucleotides. A cell may contain more than 10,000 structural genes, coding for the amino acid sequences of over 10,000 specific proteins, each gene having a sequence many hundreds of nucleotides in length. DNA consists of four different nucleotides: adenine (A); guanine (G); cytosine (C); and thymine (T). The long sequences comprising the structural genes of specific proteins are consequently very similar in overall chemical composition and physical properties. The separation of one such sequence from the plethora of other sequences present in isolated DNA cannot ordinarily be accomplished by conventional physical and chemical preparative methods.

In its native configuration, DNA exists in the form of paired linear polynucleotide strands. The strands are usually held together by interactions between an A on one strand, with a T on the opposite strand, and similarly a C on one strand and a G on the other. This is referred to as A-T and G-C base pairing. The complementary base pairing relationships described above exist between the paired strands such that each nucleotide base of one strand exists opposite its complement on the other strand. The entire sequence of one strand is mirrored by a complementary sequence on the other strand. If the strands are separated, it is possible to synthesize a new partner strand, starting from the appropriate precursor monomers (deoxynucleotide triphosphates). The sequence of addition of the monomers starting from one end is determined by, and complementary to, the sequence of the original intact polynucleotide strand, which thus serves as a template for the synthesis of its complementary partner. The synthesis of mRNA corresponding to a specific nucleotide sequence of DNA is understood to follow the same basic principle. Therefore, a specific mRNA molecule will have a sequence complementary to one strand of DNA and identical to the sequence of the opposite DNA strand, in the region transcribed. Enzymic mechanisms exist within living cells which permit the selective transcription of a particular DNA segment containing the nucleotide sequence for a particular protein. Consequently, isolating the mRNA which contains the nucleotide sequence coding for the amino acid sequence of a particular protein is equivalent to the isolation of the same sequence, or gene, from the DNA itself. If the mRNA is retranscribed to form DNA complementary thereto (cDNA), the exact DNA sequence is thereby reconstituted and can, by appropriate techniques, be inserted into the genetic material of another organism. The two complementary versions of a given sequence are therefore inter-convertible, and functionally equivalent to each other.

The nucleotide subunits of DNA and RNA are linked together by phosphodiester bonds between the 5' position of one nucleotide sugar and the 3' position of its next neighbour. Reiteration of such linkages produces a linear polynucleotide which has polarity in the sense that one end can be distinguished from the other. The 3' end may have a free 3'-hydroxyl, or the hydroxyl may be substituted with a phosphate or a more complex structure. The same is true of the 5' end. In eucaryotic organisms, i.e. those having a defined nucleus and mitotic apparatus, the synthesis of functional mRNA usually includes the addition of polyadenylic acid to the 3' end of the mRNA. Messenger RNA can therefore be separated from other classes of RNA isolated from an eucaryotic organism by column chromatography on cellulose to which is attached polythymidylic acid.

Transcription is the process by which the retrieval of information is begun. Transcription involves the resynthesis of the information in the form of RNA. Messenger RNA (mRNA) transports the information to the machinery of protein synthesis, the ribosome.

Once the mRNA is synthesized from the gene, the process of protein synthesis may begin. This process is essentially one of molecular decoding, in which the nucleotide sequence of the mRNA provides a template for the synthesis of a particular protein. Since there is a change from a nucleic acid language into that of a protein language, this process of protein synthesis appropriately is referred to as translation. As the mRNA is passed through the ribosome, groups of 3 nucleotides (codons) are positioned such as to orient accessory RNA molecules known as transfer RNA (tRNA), carrying a single amino acid into the proper alignment for the addition of the amino acid to the growing protein chain.

The coding ratio of nucleotides to amino acids is three nucleotides coding for one amino acid. Since it is necessary to code for twenty different amino acids uniquely with the available four types of nucleotides, three represents the minimum acceptable ratio. A coding ratio of one nucleotide to one amino acid would only accommodate four of the twenty amino acids necessary for protein synthesis. A coding ratio of two yielding 16 (4²) combinations likewise falls short of the required complexity. However, with a coding ratio of three, 64 (4³) different combinations are possible. This excess of twenty code words confers upon the genetic code a conditions known as degeneracy. A degenerate code contains several different code words for the same amino acid. The situation does not exist, however, where one code word would specify two different amino acids. The code may be degenerate, but it is not ambiguous.

The ability to probe the chromosome, extra chromosomal genetic material, messenger, transfer and ribosomal RNA, to synthesize genetic material, as well as to manipulate genetic material, has increased the need for means to analyze the composition and base order of genetic material. It is therefore desirable to provide for recording various genetic fragments which allow for hybridization with the complementary fragment, so that mixtures may be analyzed for the presence or absence of a particular nucleotide sequence. In the development of a system for analyzing for particular nucleotide sequences, there are many considerations. The first consideration is the ability to separate a mixture into its constituent parts, based on molecular weight and/or electrophoretic mobility. The second consideration is the ability to determine accurately the nature of the constituent parts.

One method for determining whether a particular sequence exists is hybridization. That is, a particular nucleotide sequence is marked with a detectable label, conveniently a radioactive label, and is combined with the nucleotide sequence to be analyzed. If the two sequences hybridize so as to form a strong non-covalent interaction, it may then be reasonable assumed that the sequences are substantially identical. Various techniques for accurately determining whether hybridization has occurred and for qualitatively determining the amount of the nucleotide sequence have been developed.

Another method for the detection of specific nucleic acid sequences (DNA or RNA) is achieved by hybridization of radioactive probes and autoradiography. Traditionally, radioactive probes have been produced by nick-translation, and more recently by SP6 transcription. These methods generate radioactive probes of high specific activity which are capable of detecting small concentrations of DNA or RNA sequences. However, there are several disadvantages to these methods. First, the production of probes requires the use of radioactive isotopes which have short half-lives necessitating a continuous production of fresh probes. Secondly, the labelling procedure requires the use of enzymes which are expensive and require reaction conditions which must be very carefully calibrated. Thirdly, radioactive isotopes are biologically dangerous to use. In fact, their use requires proper licensing, and their disposal is becoming increasingly expensive, difficult and hazardous.

A non-radioactive hybridization probe has been developed which utilizes biotinylated nucleotide analogues which are synthesized into probes using procedures described previously for the production of radioactive probes. Hybridization of the probes to the target sequence is detected by the interaction of biotin with avidin-conjugated enzymes, fluorescent compounds, or enzyme-linked immunodetection systems. Although the use of radioactivity is eliminated, several problems are associated with the biotin-avidin technique. One problem is sensitivity; for the most part, biotinylated probes are not as sensitive as radioactive probes. Another problem encountered is that the alteration of the nucleotides interferes with hybridization of the probe to its target.

Hybridization assays using various signal generating systems have also been developed. For example, a non-radioactive hybridization assay system which utilizes the chemical modification of nucleic acids that makes them more immunogenic has been developed. The signal is generated by reporter groups containing antibodies which recognize the altered nucleic acid. In another hybridization system, the adenine and cytosine nucleic acid bases of the probe are bonded to reporter groups by chemical modification.

The hybridization assays thus far described require the preparation of labelled probes, each of which hybridizes to a different target sequence. In order to reduce the number of labelled probes that must be prepared, a hybridization assay has been developed which utilizes a "bridging" polynucleotide between the target nucleic acid and a general signalling polynucleotide. The bridging polynucleotide consists of a single-stranded filamentous bacteriophage which contains nucleic acid sequences complementary to the target gene. The general signalling polynucleotide is a segment of single strand phage DNA which is complementary to the bridging polynucleotide.

Another problem associated with hybridization assays involves the removal of labelled probes that do not hybridize to the target sequence from the hybridization reaction because the presence of non-hybridized probes in the hybridization reaction leads to a false positive result. A hybridization assay has been provided which claims to obviate the requirement for separation of non-hybridized probes. This system requires the cohybridization of two components which, when associated, generate a signal. Another hybridization assay has been provided which utilizes photochemically-reactive intercalating agents for the covalent attachment of nucleic acids to solid supports. The immobilized target nucleic acids are capable of hybridization. Yet another immobilized hybridization assay system first involves hybridizing and then forming covalent bonds between the probe and target sequence. Immobilized sandwich hybridization techniques which require two distinct single-stranded nucleotide probes have also been reported.

Knowing the sequence of nucleotides of a messenger RNA, it is possible to write explicitly the sequence of amino acids coded therein, but the reverse is not true. Because of the degeneracy of the genetic code, a number of nucleotide sequences would be consistent with a given amino acid sequence.

In the scheme of dividing organisms with respect to the internal architecture of the cell, all cellular organisms are either prokaryotic or eukaryotic. Prokaryotes are less complex than eukaryotes in that they lack internal compartmentalization by unit membrane systems and lack a defined nucleus. Prokaryotic genetic information is carried in the cytoplasm on double-stranded, circular DNA; no other DNA is present in these cells (except for the possible presence of phage, bacterial viruses, and circular DNA plasmids, capable of autonomous replication). Eukaryotes on the other hand have a multiplicity of unit membrane systems which serve to segregate many of the functional components into specialized and isolated regions. For example, molecules storing genetic information (DNA) can be found in a well-compartmentalized nucleus and also in organelles; mitochondria and (in photosynthetic organisms) chloroplasts. The replication, transcription and translation of the eukaryotic genome occurs at either two or three distinct sites within the cell: in the nucleocytoplasmic region, in the mitochondrion and in the chloroplast.

The differences between prokaryotes and eukaryotes, however, break down when a comparison of mitochondria and chloroplasts is carried out with prokaryotes: these organelles are today considered to have been derived from free-living prokaryotes, which entered into an endosymbiotic relationship with primitive eukaryotes, and eventually became closely integrated with the machinery of the host cell and incapable of independent existence.

In this model, the eukaryotic cell is a phylogenetic "chimera" with organelle components that are clearly prokaryotic in nature. The "prokaryotic-eukaryotic" dichotomy then also has drawbacks, even as a broad classification method.

Where classification of organisms becomes more than a scientific exercise is in the identification of animals for breeding purposes. For example, the cattle breeder, or fish breeder, may wish to have a quick and reliable means of identifying different species and strains of their subjects. The correct identification of the species of these organisms is of particular importance.

A molecular approach to bacterial classification is to compare two genomes by DNA-DNA reassociation. A genetic definition of species includes the provision that strains of species are 70% or more related. With DNA-DNA reassociation, a strain can be identified only if the radioactively labelled DNA probe and unknown DNA are from the same species. The practical application of this 70% species definition, however, is limited by selection of an appropriate probe. This may be overcome in part by selecting phenotypic attributes which seem to correlate with the reassociation group; but when these are used alone, the DNA-DNA reassociation species definition is also applied indirectly. Restriction endonuclease analysis can therefore by carried out to determine common sequences in isolated genes.

The main limitation of present gene detecting methods is that they are not sensitive enough and therefore require a relatively large amount of sample to verify accurately the existence of a particular gene sequence. This is not surprising since the detection of a single gene in the entire genetic repertoire of a human being requires locating one part in one to ten million. In fact, most hybridization methods require at least one to ten micrograms of purified DNA, representing a substantial sample of cells, to perform a reliable analysis. This limitation is particularly significant in prenatal diagnosis of genetic disorders where only a small cell sample can be take, or in identifying infectious agents such as viruses in small tissue samples. Consequently, there is a substantial need for gene detection methods which are more sensitive than the hybridization assay and which provide more information.

A series of similar individuals showing certain common features that are recognizably different from other such series is referred to as a species. Thus, members of a particular species may be identified because they exhibit certain defined characteristics or traits. Although these characteristics are usually morphological, there is an underlying genetic basis for them. The traits are maintained by the genetic structure of the species. Members of the species can breed successfully with other members of the species, but do not do so with members of other species. This means that individuals within a species will share a specific set of genetic markers that may be used to define that particular species.

There is also variation among members of species. This variation may be restricted to a single individual and its immediate relatives, or it may be characteristic of a subset of the species as a whole. A subset may constitute a subspecies or population (i.e. a group of individuals within a species who confine their breeding to other members of that group although they are capable of breeding with any member of the species). The sum of the genetic content of all the populations in a species forms the gene pool of that species. Therefore, the genetic material of an organism should comprise: (a) segments that are unique to themselves (individual identifiers); (b) segments that are shared by other individuals that are part of its subspecies or population (population identifiers); (c) segments that are found in every member of the species but not in other related species (species identifiers); and (d) components that are found in many different related species. By judicious use of these different regions of a species' genetic material, it should be possible to determine the species and/or population origin of an individual.

There is a long history of using protein markers for identifying different species. However, proteins have several shortcomings in this respect. For example, they vary from tissue to tissue and therefore cannot be used interchangeably when one has samples of hair, skin, blood and muscle to examine. Changes to proteins may occur after death or during food-processing (e.g. even upon freezing and thawing). In addition, there is a loss of information content as one goes from DNA to protein and it is not always possible to use proteins to tell closely related species apart.

Identity is generally defined as "sameness in all that constitutes the objective reality of a thing". Though the primary concern is with the identification of the species and/or population of an organism, the problem of how best to carry out identification arises in those situations in which one must determine whether an unknown object, in all essential respects, matches a particular known object lying within a large collection of diverse known objects. In the context of biology, the term identification, as used herein, encompasses the determination of whether an unknown organism, or tissue derived therefrom, falls within a particular group of organisms constituting a species or a subset of that species, i.e. a population.

The logical approach to the problem of identification is to find an identifier or identifiers that are shared by all members of a species and/or population but are absent from other species. The genetic information, or DNA, of an individual contains segments that can be used as species and/or population identifiers.

Canadian Patent No. 1,215,304, patented December 16, 1986 by J. Glassberg, attempted to solve one particular problem relating to the determination of paternity in sexually reproducing organisms and to establish individual genetic identity. These objectives were said to be achieved by analyzing the DNA of the organism in respect to one or more polymorphic genetic regions, differentiating the polymorphisms in terms of relative size of the genetic regions and by so doing, characterize an individual member of the species. It was also taught that the polymorphic genetic regions could be detected by the following steps: isolating the DNA of the individual to be analyzed; subjecting that DNA to the action of restriction endonucleases; sizing and converting DNA fragments generated as described above to single-stranded molecules; hybridizing the sized, single-stranded molecules with probe DNA molecules and identifying the number and location of the hybridized fragments. The probe molecules were those which had been generated by endonuclease digestion of genomic DNA.

It was also taught, by that patentee, that the polymorphic genetic regions could be detected by the following steps: isolating the DNA of the individual to be analyzed; subjecting the DNA to the action of restriction endonucleases; sizing and converting DNA fragments generated as described above to single-stranded molecules; hybridizing the sized, single-stranded molecules with probe DNA molecules; and identifying the number and location of the hybridized fragments. The probe was one that did not hybridize to the human HLA genetic locus.

However, this technique did not provide for the determination of the identify of the genus, and within a particular genus, the identify of the species and/or strain and/or subset and/or sub species of that particular species of an organism (sample) and/or the population identity of that organism (sample) by being able to define the nucleotide sequences of segments of genomes.

Canadian Patent No. 1,214,980, patented December 9, 1986 by J.A. Webster, Jr., was alleged to provide a method of objectively identifying organisms which utilized the organisms' genomes. The patent provided a method of characterizing an unknown organism which comprised comparing the electrophoretic pattern of restriction endonuclease-digested DNA from the organism, which digested DNA had been hybridized or reassociated with ribosomal RNA information-containing nucleic acid from or derived from a probe organism, with equivalent electrophoretic patterns of at least two known different organism species. Thus, the patentee specifically provided a method of characterizing an unknown organism by the steps of: comparing the electrophoretic pattern of restriction endonuclease-digested DNA from an unknown organism, the digested DNA having been hybridized or reassociated with ribosomal RNA information-containing nucleic acid from or derived from a known probe organism, with at least two equivalent electrophoretic patterns, each one of the equivalent electrophoretic patterns defining a known different organism species; and establishing the species of the unknown organism by means of a conserved set of ribosomal RNA sequence-containing restriction fragments present in the electrophoretic pattern of the unknown organism.

That patentee also provided a method for detecting a prokaryotic organism while in the presence of, or associated with, a eukaryotic organism which comprised: selectively hybridizing ribosomal RNA sequences of the prokaryotic organism with a detectably-labelled prokaryotic rRNA information containing hybridization probe.

That patentee still further provided a kit comprising a carrier: compartment in the carrier to receive in close confinement therein one or more containers, a first container containing ribosomal RNA (rRNA) information-containing nucleic acid from, or derived from, a probe organism; and a catalogue having electrophoretic band patterns of rRNA information-containing restriction fragments for at least two known different organism species, or DNA derived therefrom. The kit could also include a second container containing one or more restriction endonuclease enzymes.

However, this technique did not provide for the determination of the identify of the genus, and within a particular genus, the identify of the species and/or strain and/or subset and/or sub species of that particular species of an organism (sample) and/or the population identity of that organism (sample) by being able to define the nucleotide sequences of segments of genomes.

Canadian Patent No. 1,221,298, patented May 5, 1987 by J.A. Webster, Jr., provided a method of characterizing an unknown organism species by determining the position of part or whole of evolutionarily-conserved sequences in genetic material of the organism, relative to a known position of restriction endonuclease cleavage sites in the genetic material (other than by determining the electrophoretic pattern of restriction endonuclease digested DNA from the unknown organism, which digested DNA has been hybridized or reassociated with ribosomal RNA information-containing nucleic acid from or derived from a known probe organism), thereby to obtain an identifying genetic characterization of the unknown organism, and then comparing that characterization with information from at least two sets of identifying genetic characterizations derived from the same conserved sequences, each of the sets defining a known organism species.

That patent also provided a kit comprising: a carrier; compartments therein to receive in close confinement therein one or more containers, in which a first container contained conserved genetic material sequence information-containing nucleic acid (other than ribosomal RNA information-containing nucleic acid) from, or derived from, a probe organism or from a consensus sequence; and containing a catalogue having hybridized or reassociated electrophoretic band patterns for at least two known different organism species, or genetic material derived therefrom. That kit could also include a second container containing one or more restriction endonuclease enzymes.

However, this technique did not provide for the determination of the identify of the genus, and within a particular genus, the identify of the species and/or strain and/or subset and/or sub species of that particular species of an organism (sample) and/or the population identity of that organism (sample) by being able to define the nucleotide sequences of segments of genomes.

U.S. Patent No. 4,283,489, patented August 11, 1981 by H.M. Goodman et al., provided a method for purifying a specifically desired DNA sequence, starting from RNA heterogeneous in length and sequence. The method employed restriction endonuclease cleavage of cDNA transcribed from a complex mixture of mRNA. The method made use of transcription of RNA into cDNA, the sequence specific fragmentation of this cDNA with one or two restriction endonucleases, and the fractionation of the cDNA restriction fragments on the basis of their length. The use of restriction endonucleases eliminated size heterogeneity and produced homogeneous length DNA fragments of desired sequences. The length of the fragments depended on the number of nucleotides separating the restriction sites and will usually be different for different regions of DNA. Fractionation by length enabled purification of a homogeneous population of fragments having the desired sequence. The fragments were homogeneous in size and highly pure in terms of nucleotide sequence.

Specific sequences purified by the procedure outlined above could be further purified by a second specific cleavage with a restriction endonuclease capable of cleaving the desired sequence at an internal site. This cleavage resulted in formation of two sub-fragments of the desired sequence, separable on the basis of their length. The sub-fragments were separated from uncleaved aid specifically cleaved contaminating sequences having substantially the same original size. Because of the rarity and randomness of placement of restriction endonuclease recognition sites, an extremely low probability that a contaminant having the same original length will be cleaved by the same enzyme to yield fragments having the same length as those yielded by the desired sequence. After separation from the contaminants, the sub-fragments of the desired sequence may be rejoined using techniques known in the art, to reconstitute the original sequence. The two sub-fragments must be prevented from joining together in the reverse order to their original sequence.

However, this technique did not provide for the determination of the identify of the genus, and within a particular genus, the identify of the species and/or strain and/or subset and/or sub species of that particular species of an organism (sample) and/or the population identity of that organism (sample) by being able to define the nucleotide sequences of segments of genomes.

U.S. Patent No. 4,302,204, patented November 24, 1981 by G.M. Wahl et al., provided techniques for the transfer and detection of separated nucleic acids, both RNA and DNA. The patentee provided for the preparation of resolved nucleotide sequences covalently affixed to a stable substrate, which are then used for hybridization with nucleotide sequences for determination of the presence of a complementary nucleotide sequence. In an initial phase, a particular nucleotide sequence was prepared for transfer from a source of the sequence to a chemically-reactive substrate, e.g. diazo substituted paper, to affix the nucleotide sequence to the substrate by covalent bonding of the chemically reactive functionality to the polynucleotide, to provide for storage stability. When the nucleotide sequence had been previously subjected to resolution, particularly a resolution based on molecular weight and electrophoretic mobility, the position of the nucleotide sequence on the paper was related to its chemical composition and molecular weight. Once the nucleotide sequence was transferred from the resolving medium to the substrate, and covalently affixed thereto, the substrate may now be used for probing compositions having unknown nucleotide sequences to determine the presence of a sequence complementary to the affixed nucleotide sequence. A hybridization buffer was employed including a volume exclusion or renaturing agent, which greatly enhances the rate and efficiency at which a complementary nucleotide strand hybridizes to the affixed strand.

Prior to hybridization, DNA was treated differently from RNA. Depending upon the molecular weight of the DNA during electrophoresis, differing mixtures are employed to enhance resolution. Where relatively low molecular weight DNA was involved, cross-linked polymers were employed to provide for a hard polymer, where the cross-links therein were susceptible to cleavage without adverse affects on the DNA. Where the DNA was of a size in excess of 200 bases in length, the DNA was subjected to successive treatments of depurination and then, degradation and denaturation, so as to provide for randomly-formed single-stranded smaller fragments. The nucleotide sequences to be assayed may be labelled, particularly with a radioactive marker. After hybridization, the presence of the radioactively-marked nucleotide sequences may be determined by autoradiography. In this manner, the presence or absence of a particular sequence can be determined, as well as a quantitative evaluation of its amount.

However, this technique did not provide for the determination of the identify of the genus, and within a particular genus, the identify of the species and/or strain and/or subset and/or sub species of that particular species of an organism (sample) and/or the population identity of that organism (sample) by being able to define the nucleotide sequences of segments of genomes.

U.S. Patent No. 4,358,535, patented November 9, 1982 by S. Falkow et al., provided specific DNA probes for use in diagnostic microbiology. In carrying out the patented method, a clinical isolate suspected of containing the pathogen may be used directly or cultivated under conditions where clones are grown providing high multiplication of the pathogenic organism. After treating the genome to provide single-stranded genomic nucleic acid and fixing the nucleic acid to a support, the affixed DNA or RNA was contacted with a labelled polynucleotide having a base sequence complementary to the coding or antisense strand of a gene coding for a product characteristic of the pathogen.

The primary reagent was the labelled probe. The probe may be RNA or DNA. The probe will normally have at least 25 bases, more usually at least 30 bases, and may have up to 10,000 bases or more, usually having not more than 5,000 bases. The probe sequence will be at least substantially complementary to a gene coding for a product characteristic of the pathogen, usually a cytoplasmic product or released product, particularly an excreted product. The probe need not have perfect complementarity to the sequence to which it hybridizes; there may be 30% or more of mismatched pairs.

However, this technique did not provide for the determination of the identify of the genus, and within a particular genus, the identify of the species and/or strain and/or subset and/or sub species of that particular species of an organism (sample) and/or the population identity of that organism (sample) by being able to define the nucleotide sequences of segments of genomes.

U.S. Patent No. 4,526,865, patented July 2, 1985 by R. Silman, provided a microorganism identification technique. The patented method for identifying an unknown microorganism included the steps of: preparing a specimen of the microorganism to which a radioactive emissive agent has been added that is actively incorporated into the products of metabolism of the microorganism to produce a mix of radioactive peptide or protein-emissive products in a manner that depends on the metabolic mechanism of the microorganism; separating the peptide or protein emissive products in the mix resulting from the preparation of a specimen; detecting the separated peptide or protein-emissive products to derive a characteristic pattern therefrom that depends on the conditions under which the specimen is prepared, which conditions are standardized to provide a repeatable characteristic pattern functioning as a unique identifier for the unknown microorganism; and comparing the identifier for the unknown microorganism with stored information relating to the characteristic patterns of known microorganisms to determine the identification of the unknown.

However, this technique did not provide for the determination of the identify of the genus, and within a particular genus, the identify of the species and/or strain and/or subset and/or sub species of that particular species of an organism (sample) and/or the population identity of that organism (sample) by being able to define the nucleotide sequences of segments of genomes.

U.S. Patent No. 4,882,269, patented November 21, 1989 by R.J. Schneider et al., provided an amplified hybridization assay. The patented invention provided a hybridization assay that generated an enormously amplified signal upon hybridization to the target sequence of interest. Accordingly, a primary probe was provided, a small segment of which hybridized to the target DNA of interest. A family of signal-generating secondary probes was also provided which provided for an enormous amplification of the signal generated by the hybridization event. Depending upon the configuration of the assay components, the hybridization event may occur between mobile species or between combinations of fixed and mobile species. Thus, the patentee provided a method for the detection of a target nucleotide sequence, comprising: contacting the target nucleotide under conditions that permit hybridization with a primary probe which comprised a polynucleotide sequence and a polymeric tail that had binding sites that were incapable of binding to the target sequence, and a plurality of secondary probes comprising a family of signal-generating probes, each member of which comprised a signal-generating component and a polymer capable of binding to a different portion of the tail of the primary probe; and detecting the amplified signal generated by a previously-formed reaction product, in which the polynucleotide sequence of the primary probe was hybridized to the target nucleotide and a plurality of secondary probes were bound to different portions of the primary probe tail. The patentee also provided a hybridization assay kit for the detection of a target nucleotide sequence, comprising: a primary probe which comprised a polynucleotide sequence that is complementary to the target nucleotide sequence and a polymeric tail that had binding sites that were incapable of binding to the target sequence; and a plurality of secondary probes comprising a family of signal-generating probes, each member of which comprised a signal-generating component and polymer capable of binding to a different portion of the tail of the primary probe, which provided for the generation of an amplified signal when the polynucleotide sequence of the primary probe is hybridized to the target nucleotide and the plurality of secondary probes are bound to a different portion of the primary probe tail.

However, this technique did not provide for the determination of the identify of the genus, and within a particular genus, the identify of the species and/or strain and/or subset and/or sub species of that particular species of an organism (sample) and/or the population identity of that organism (sample) by being able to define the nucleotide sequences of segments of genomes.

U.S. Patent No. 4,908,307, patented March 13, 1990 by K.D. Rodland et al., provide a hybridization probe and method for detecting nucleotide sequences. Initially, a DNA sample of interest was purified chemically and cut into pieces with a suitable restriction enzyme. The pieces were separated by size by electrophoresis in a suitable gel. The pieces of interest were then typically transferred to an immobilizing medium, e.g. a nitrocellulose or nylon-base membrane, that retained the geometry of the pieces. The membrane was thereafter dried and prehybridized to equilibrate it for later immersion in a hybridization solution.

A probe was constructed from a nucleotide sequence complementary to the gene sample by a nick translation reaction, using both a DNase and DNA polymerase. The probe and sample are thereafter combined in a hybridization buffer and incubated. In such buffer, the probe and sample are combined in the absence of reducing agents, nonpolar solvents and dextran sulfate. After a specified incubation period, the membrane is removed from the buffer and washed free of extraneous materials. The presence or absence of the particular nucleotide sequence is detected by autoradiography and quantified by liquid scintillation counting.

However, this technique did not provide for the determination of the identify of the genus, and within a particular genus, the identify of the species and/or strain and/or subset and/or sub species of that particular species of an organism (sample) and/or the population identity of that organism (sample) by being able to define the nucleotide sequences of segments of genomes.

Mullis et al., U.S. Patent No. 4,683,195 patented July 28, 1987 provided a teaching of the polymerase chain reaction (PCR) process for amplifying, detecting, and/or cloning nucleic acid sequences. According to the patent, the process comprised treating separate complementary strands of the nucleic acid with a molar excess of two oligonuleotide primers, extending the primers to form complementary primer extension products which acted as templates for synthesizing the desired nucleic acid sequence, and detecting the sequence so amplified. The steps of the reaction could be carried out stepwise or simultaneously and could be repeated as often as desired.

In addition, a specific nucleic acid sequence could be cloned into a vector by using primers to amplify the sequence, which contained restriction sites on their non-complementary ends, and a nucleic acid fragment could be prepared from an existing shorter fragment using the amplification process. A complete copy of this patent is attached to this patent application. The entire content of this patent is incorporated herein by reference.

By using the PCR process, unique sequences could be cloned in vitro in a matter of hours. Furthermore, the procedure was easily automated, so that hundreds of samples could be amplified each day. The enzymatic amplification of a specific DNA segment was made possible by the highly specific binding of oligonucleotide primers to sequences flanking the segment. These primers allowed the binding of a DNA polymerase that then copied the segment. Because each newly made copy can serve as a template for further duplication, the number of copies of the target segment grew exponentially.

A description of the polymerase chain reaction was also given in the October 1, 1990 issue of "Chemical and Engineering News". As described therein, PCR was an enzymatic process that was carried out in discrete cycles of amplification, each of which could double the amount of target DNA in the sample. Thus, n cycles can produce 2ⁿ times as much target as was present to begin with. It was not necessary to know the nucleotide sequence of a target in order to amplify it using PCR, but the sequence of a small stretch of nucleotides on each side of the target must be known. These flanking sequences were used to design two synthetic single-stranded oligonucleotides, usually 20 nucleotides in length, that could serve as primers. The sequence of these primers was chosen so that each had base pair complementarity with its respective flanking sequence.

PCR begins by denaturing the double-stranded target DNA, followed by annealing the primers (one for each strand) to the sequences flanking the target. Each primer formed a duplex with its flanking sequence so that the 3'hydroxyl end of the primer faced the target sequence. Addition of DNA polymerase and deoxynucleoside triphosphates caused a new DNA strand to form beginning at the primer and extending across the target sequence, thereby making copies of the target. These steps - DNA denaturation, primer annealing, and DNA polymerase extension - represent one PCR cycle; each step was carried out at an appropriate temperature.

If the extension product of a primer was long enough, it would include the sequence complementary to the primer at the other end of the target sequence. Thus, each new extension product could act as a template for the next cycle. It is this fact, first recognized by Mullis, that lead to the exponential increase in PCR product with each cycle. If the amount of target exactly doubles with each cycle, as few as 20 cycles will generate about a million times more target sequence than originally was present.

Amplification by PCR was extremely rapid. Twenty-five cycles could be carried out in just over one hour. Beginning with 1»g of total human DNA, which contained 300,000 copies of each unique sequence, 25 cycles of PCR could generate up to several micrograms of a specific product (on the order of a few picomoles). The size of the final product was equal to the sum of the lengths of the two primers and the length of the target sequence that stretched between them. PCR products up to several thousand base pairs long could be routinely synthesized.

The scientific literature has also provided some disclosure relevant to some extent to the present invention.

Meyer et al., "MITOCHONDRIAL GENES AMPLIFIED VIA THE POLYMERASE CHAIN REACTION INFERENCES FOR THE EVOLUTION OF CICHLID FISHES MITOCHONDRIAL GENE SEQUENCES". Annual Meeting of the American Society of Zoologists, American Microscopical Society, Animal Behaviour Society, The Crustacean Society, International Association of Astacology Society of Systematic Zoology, and the Western Society of Naturalists, San Francisco, California, U.S.A., December 27-30, 1988. AM ZOOL 28 (4). 1988. 35A. merely described the use of PCR/direct sequencing to analyze the genetic relationships among a group of fish. According to this paper, a fast method for sequencing of DNA was developed and applied to mitochondrial genes of cichlids. Parts of the 12S rRNA and the cytochrome-b gene were amplified via the polymerase chain reaction (PCR). The amplification products were sequenced directly by the dideoxy technique. A total of 20 taxa from Africa and the Neotropics were examined and their phylogenetic relationships determined. The rate of sequence divergence was found to be about twice as fast in the cytochrome-b as in the 12S rRNA gene.

KOCHER et al., "AMPLIFICATION AND DIRECT SEQUENCING OF DIVERGENT MITOCHONDRIAL DNAS VIA THE POLYMERASE CHAIN REACTION". Colloquium On Molecular Evolution held at the 18th Annual UCLA (University of California-Los Angeles) Symposia On Molecular and Cellular Biology, Lake Tahoe, California, US, February 27-March 6, 1989. J Cell Biochem Suppl O (13 part c). 1989. 120, taught that primers that amplified three regions of human mitochondrial DNA via the polymerase chain reaction also amplified the corresponding regions from 99 other species, including mammals, birds, amphibians, fishes, and some invertebrates. Amplification and direct sequencing were possible using unpurified mtDNA from nanogram samples of fresh specimens and microgram amounts of tissues preserved for months in alcohol or decades in the dry state. These sequences provided a consistent metric for phylogenetic comparisons ranging from the population to the interclass level in vertebrates.

Meyer et al., "MITOCHONDRIAL GENES AMPLIFIED VIA THE POLYMERASE CHAIN REACTION DYNAMICS OF NUCLEOTIDE SUBSTITUTIONS AND EVOLUTION OF CICHLID FISHES", Colloquium On Molecular Evolution held at the 18th Annual UCLA (University Of California-Los Angeles) Symposia On Molecular and Cellular Biology, Lake Tahoe, California, USA, February 27-March 6, 1989. J Cell Biochem Suppl O (13 part c). 1989. 132, taught that parts of the mitochondrial 12S rRNA and the cytochrome-b genes of cichlid fishes could be amplified with human primers and sequenced directly. 20 taxa of cichlid fishes from Africa and the Neotropics were examined and their phylogenetic relationships investigated. Sequence divergence was found to be about twice as fast in the cytochrome-b as in the 12S rRNA gene. The evolutionary patterns of sequence divergence was found to be the same in these morphologically fast evolving fishes, as it was for published cases of mammals. In the cytochrome-b gene, it was found that there was a strong transition bias for closely related taxa and C-T changes outnumbered G-A by far. Replacement changes were rare, amino acid changes matched predictions based on the function of cytochrome-b. The 12S rRNA sequences diverged at faster rates in loops than in stem regions.

Kocher et al., "DYNAMICS OF MITOCHONDRIAL DNA EVOLUTION IN ANIMALS AMPLIFICATION AND SEQUENCING WITH CONSERVED PRIMERS", Proc Natl Acad Sci USA 86 (16). 1989. 6196-6200, described the use of the polymerase chain reaction to amplify homologous segments of mtDNA from more than 100 animal species, including mammals, birds, amphibians, fishes, and some invertebrates using a standard set of primers directed toward conserved regions. Amplification and direct sequencing were possible using unpurified mtDNA from nanogram samples of fresh specimens and microgram amounts of tissues preserved for months in alcohol or decades in the dry state. The bird and fish sequences were found to have evolved with the same strong bias toward transitions that holds for mammals. Amino acid replacement in a segment of the cytochrome-b gene was found to be faster in mammals and birds than in fishes and the pattern of replacement fit the structural hypothesis for cytochrome-b. Thus, the paper described methods and primers, the method being described to obtain the sequences from known specimens for evolutionary studies. The intent of the authors was an unstated implication that if one knew the sequence one could determine the species.

Jayaraman et al., "PCR MEDICATED GENE SYNTHESIS" Nucleic Acids Res. 1989 Volume: 17 Number: 11, page: 4403, described a method for gene synthesis which involved a combination of a single-step ligation of oligonucleotides and PCR amplification of the crude ligation mixture. This procedure enabled the synthesis of tuna and horse cytochrome C genes.

Whitmore et al., "RFLP ANALYSIS OF FISH MITOCHONDRIAL DNA AMPLIFIED BY THE POLYMERASE CHAIN REACTION", Annual Meeting of the American Society of Zoologists, American Microscopical Society, Animal Behaviour Society, the Crustacean Society, International Association of Astacology, and the Society of Systematic Zoology, San Antonio, Texas, USA, December 27-30, 1990. Am Zool 30(4), 1990, 72A described non-invasive genetic analysis of fish mitochondrial DNA genes, which performed by isolating DNA from the epidermis covering a few scales. The polymerase chain reaction (PCR) was used to amplify selected sequences, including portions of the 12S rRNA, cytochrome-b and ATPase 6 genes. Interspecific and intraspecific variation in several centrarchid species was examined both by restriction endonuclease digestion of the PCR products and allele-specific oligonucleotide probes (ASOPs). Species were readily identified by RFLP profiles of amplified sequences. Maternal lineage of hybrids could also be readily deduced from RFLP profiles. This paper thus described a method using PCR/direct sequencing to identify fish species. No details were given, but it could be inferred that the methodology was an extension of the Kocher et al. papers described above.

Block et al., "EVOLUTION OF ENDOTHERMY IN SCOMBROID FISHES: SYSTEMATIC RELATIONSHIPS EXAMINED WITH DIRECT SEQUENCING OF MITOCHONDRIAL DNA". Annual Meeting Of The American Society of Zoologists, American Microscopical Society, Animal Behaviour Society, The Crustacean Society, International Association of Astacology, And The Society of Systematic Zoology, San Antonio, Texas, USA, December 27-30, 1990. Am Zool 30 (4). 1990. 72A, described the relationships of the five families of fishes comprising the suborder Scombroidei using mtDNA. This paper described the amplification and sequencing of the mitochondrial cytochrome-b in Scombrids (tunas). They looked at 15 taxa (no species names given). No sequence data or methods are described.

Carr and Marshall, "DETECTION OF INTRASPECIFIC DNA-SEQUENCE VARIATION IN THE MITOCHONDRIAL CYTOCHROME-B GENE OF ATLANTIC COD (GADUS-MORHUA) BY THE POLYMERASE CHAIN-REACTION". Canadian Journal of Fisheries and Aquatic Sciences, 1991, V48, N1 Pages 48-52, described the determination of the DNA sequence of a portion of the mitochondrial cytochrome-b gene for 55 Atlantic cod (*Cadus morhua*) from Norway and from ten locations within the Northern Cod complex and adjacent stocks off Newfoundland. DNA was prepared for sequencing by the polymerase chain reaction (PCR). This paper thus described using PCR/direct sequencing to look at the population structure of cod stocks. It did not use the technique for species identification.

Journal of General Microbiology, vol. 136, Part 9, pages 1915-1920, "Differentiation of mycobacterium species by direct sequencing of amplified DNA's, ROGALL et al., described a method for the identification of Mycobacterium species by direct sequencing of amplified DNA which can then be compared with known species-specific rRNA sequences.

A description of the present invention has been published after the priority date of the present application in a paper by BARTLETT and DAVIDSON, "IDENTIFICATION OF *THUNNUS* TUNA SPECIES BY THE POLYMERASE CHAIN-REACTION AND DIRECT SEQUENCE-ANALYSIS OF THEIR MITOCHONDRIAL CYTOCHROME-B GENES" in Canadian Journal of Fisheries and Aquatic Sciences, 1991, V48, N2, P309-317. Thus, that paper described the forerunner of the present invention and has been incorporated into the present specification.

### (iii) Disclosure of the Invention

The technical problem to be solved is that the use of PCR technique in the prior art as discussed above did not provide for the determination of the species' identity of the species of an organism (sample) and/or the population identity of that organism (sample) by being able to define the nucleotide sequences of segments of genomes. Accordingly, there presently exists a need for a rapid, accurate and reliable means for identifying species and subspecies. Further, a need also exists for a method useful for determining the genus, species, strain, sub-species, sub-sets or population origin of an individual organism or tissue derived from that organism. The method should be generally and readily useful in diagnostic laboratories. It should not be dependent on the number of tests done, on the subject prejudices of the persons carrying out the tests, nor the fortuitous or unfortuitous trial and error methods of the past.

Thus the technical problem to be solved is to provide a method which can quickly, reliably, accurately and objectively determine the genus, species, strain, sub-species, sub-sets or population identity of an organism or tissue derived from that organism by means which utilizes the organism's genome. This technical problem may also be characterized as being to provide a method of analysis to define segments of genomes which may be used to determine genus, species, strain, sub-species, sub-sets or population identity.

The present invention generally provides a method for determination of the genus, and then the species, strain, sub-species or sub-set of sample of a eukaryote or a prokaryote organism, or for the determination of the species origin of a sample of an organism or the population identity of said organism, characterized by the steps of:
- isolating DNA from said sample;
- amplifying a defined segment of said DNA;
- first determining if there is a match of the DNA sequence of said sample with any DNA sequence in a data base of DNA sequences from known species by comparing that DNA sequence with said data base to establish the identity of said sample;
- then if no direct match is observed, carrying out a cladistic analysis to determine the closest species by the additional steps of: extracting DNA from a close genus, species, strain, sub-species or sub-set; amplifying and sequencing segments of interest therefrom; and comparing that sequenced segment with the sequence from the sample; and repeating said above-defined additional steps until a match is found and the identity of the sample established.

This invention also provides a kit comprising a carrier; compartments within said carrier to receive one container containing DNA from a defined eukaryote or prokaryote organism; a second container containing an amplified segment of the DNA from said defined organism; and a third container containing DNA sequences for at least one of a plurality of genera, species, strain, sub-species, sub-set, and DNA derived therefrom.

The advantageous effect of this invention is that it provides a procedure for identifying the animal origin of biological specimens, namely, Forensically Informative Nucleotide Sequencing (FINS). FINS is composed of four steps. FINS is used to identify the species of origin of a specimen, according to this invention, by providing conditions that allow each step to be carried out routinely and reproducibly. The steps are as follows: first, DNA is extracted from the biological sample; second, a specific segment of the isolated DNA is amplified; third, the nucleotide sequence of the amplified DNA is determined; and fourth, a phylogenetic analysis is carried out on the nucleotide sequence to determine its closest relative(s) in a data base.

The advantageous effect of this invention, relies on the ability to isolate DNA from the sample in question; the amplification of a defined segment of the DNA; the determination of the DNA sequence of the amplified segment; the comparison of this DNA sequence with a data base of sequences from known species; and a cladistic analysis of these sequence data.

The method of one mode of this invention is characterized by the steps of first determining if there is a match of the DNA sequence of a sample with any DNA sequence in the data base. If no direct match is observed, a cladistic analysis is carried out to determine the closest genus, species, strain, sub-species, or sub-sets. DNA is extracted from these genera, species, strains, sub-species, or sub-sets. The segments of interest are amplified and sequenced and then compared with the sequence from the sample. These steps are repeated until a match is found and the identity of the sample established. The amplification of such DNA is preferably carried out using primer cyt bH 5'CCCCTCAAATGATATTTGTCCTCA3' and primer cyt bL 5'CCATCCAACATCTCAGCATGATGAAA3', respectively.

This method may be carried out to distinguish mammals, birds, and fish in freshly frozen samples; or to distinguish between closely related fish, namely Atlantic salmon and brown trout; or to distinguish between domesticated animals, namely cow, pig, sheep, goat and horse; or to distinguish between poultry, namely chicken and turkey; or to distinguish between game, namely deer, moose and caribou; or to distinguish between salmon and mackerel in smoked samples; or to distinguish between herring species in pickled samples; or to distinguish between salmon and tuna species in canned samples; or to distinguish between cod species in salted samplesl or to distinguish between battered cod and chicken nuggets in partially cooked samples; or to distinguish between various birds in blood smears in side samples; or to distinguish between various mammals and birds in preserved skin samples.

The method of another mode of the invention is characterized by the steps of: isolating DNA from the sample; amplifying a defined segment of the DNA; determining the nucleotide sequence of the amplified segment; and comparing that DNA sequence with a data base of DNA sequences from known species to establish the identity of the sample, which preferably includes the further steps of: first determining if there is a match of the DNA sequence of the sample with any DNA sequence in the data base; and if no direct match is observed, carrying out the cladistic analysis to determine the closest species; by the steps of: extracting DNA from the genus, species, strain, sub species or sub-set; amplifying and sequencing the segments of interest, and comparing the sequences with the sequence from the sample; and repeating these steps until a match is found and the identity of the sample established.

The amplification of such DNA is carried out using primer cyt bH 5'CCCCTCAAATGATATTTGTCCTCA3' and primer cyt bL 5'CCATCCAACATCTCAGCATGATGAAA3', respectively.

In one specific feature of this invention, the polymerase chain reaction was used to amplify a 307 base pair segment of the mitochondrial cytochrome-b gene from members of four species of tuna. There is intraspecific variation at this locus in each of the species. More importantly, there are differences between the four species and these genetic markers can be used to determine the species identity of an individual tuna with a high degree of confidence. Thus, the method may be used to distinguish the following species of tuna by the presence of nucleotide at specific sites according to the following table:

| | Position | | | | | |
|---|---|---|---|---|---|---|
| Species | 35 | 62 | 68 | 89 | 227 | 260 |
| Bluefin | G | T | T | T | G | C |
| Bigeye | T | T | T | T | G | T |
| Yellowfin | G | C | T | T | G | T |
| Albacore | G | T | C/G | G | A | T |

The DNA is preferably isolated from the sample by: homogenizing tissue for which DNA is to be isolated; extracting the homogenate; separating the phases by centrifugation; precipitating the DNA from the aqueous phase with an alcohol; collecting the DNA as a pellet; and using the crude total mitochondrial and genomic DNA in the pellet. The homogenizing step preferably is carried out in an aqueous solution containing guanidinium thiocyanate, sodium citrate, sarcosyl and mercaptoethanol. The extraction step preferably is carried out with a mixture of phenol: chloroform:isoamyl alcohol. The collecting of the DNA is preferably carried out by: separating the phases by centrifugation; adding an equal volume of isopropanol to the aqueous phase; cooling to -20°C; and centrifuging to pellet the DNA. The DNA preferably is obtained by the steps of: washing the pellet with cold ethanol; drying the DNA pellet; and dissolving the dried pellet in a solution of Tris/HC1 and EDTA.

Crude mitochondrial or genomic DNA may be amplified by: first chemically synthesizing two oligonucleotides that flank the defined segment of the genome to be amplified; using these oligonucleotides as primers in a series of cycles which include separating double-stranded DNA, annealing the primers to the template DNA, and elongating the defined segment of DNA using a heat-stable DNA polymerase and deoxynucleotide triphosphate precursors; electrophoresing the reaction mixture, staining the DNA and excising the amplified segment of DNA from the gel; preparing a single-stranded template by asymmetric amplification; removing residual nucleotides, primers and buffer salts from the single-stranded DNA by centrifugal dialysis; and recovering the single-stranded DNA. The amplification reaction preferably is carried out in an aqueous solution containing Tris/HCl, magnesium chloride; 2-mercaptoethanol; dATP; dCTP; dGTP; dTTP; each primer; and a heat-stable, prokaryotic DNA polymerase, e.g. by about 30 to about 40 cycles of: about 92°C for about 45 seconds as the denaturation step, about 50°C for about 45 seconds as the annealing step, and about 72°C for about 90 seconds as the extension step. The excising of the DNA is preferably carried out by first electrophoresing the reaction mixture in a low melting agarose gel made with a Tris, sodium acetate, buffer; visualising the DNA by staining with ethidium bromide and excising the amplified segment of DNA from the gel and melting in water. The single-stranded template preferably is prepared by asymmetric amplification in an aqueous solution containing Tris/HCl, magnesium chloride, 2-mercaptoethanol, dATP, dCTP, dGTP, dTTP, each primer, and a heat-stable, prokaryotic DNA polymerase. The amplification is preferably carried out for about 30 to about 40 cycles of about 92°C for about 45 seconds, about 50°C for about 45 seconds, and about 72°C for about 90 seconds.

By yet another embodiment and feature, the DNA sequencing of the single-stranded amplified product is carried out using the dideoxy chain termination DNA sequencing procedure. The limiting primer used in the asymmetric single-stranded amplification reacting is preferably used as the sequencing primer. The analysis routinely covers at least 300 nucleotides of the defined segment.

Specific complementary sequences to characterize species of tuna are: part of the cytochrome-b gene of THUNNUS TUNA having the following sequence: 
                                  *     * **
AGCCAACGAA   GGCGGTCATC   ATAACTAGGA   GTAGGAGTAC   40
                           *     *        *
TACTCCGATG   TTTCATGTTT   CTTTGTATAG   GTAAGAGCCG   80
        *           *              *     *
TAGTAAAGTC   CTCGGCCGAT   GTGGAAGTAG   ATRCAGATAA   120
                *                          *  *
AGAAGAAAGA   GGCCCCGTTT   GCGTGGAGGT   TCCGGATGAG   160
   *     *                                      *
TCAACCGAAG   TTGACATCTC   GGCAAATGTG   GGCTACTGAG   200
                 *              *
GCGAAGGCTG   ATTCGACATC   AGGGGTGTAG   TGTATTGCGA   240
                      *    *
GGAATAGTCC   TGTAAGGATT   TGAGAAATAA   GGCAAAGGCC   280
AAGTAGTGAG   CCAAAGTTT                              299;
part of the cytochrome-b gene of BLUE FIN TUNA haying the following sequence: 
AGCCAACGAA GGCGGTCATC ATAACTAGGA GTAGGAGTAC 40
TACTCCGATG TTTCATGTTT CTTTGTATAG GTAAGAGCCG 80
*
TAGTAAAGTC CTCGGCCGAT GTGGAAGTAG ATACAGATAA 120
*
AGAAGAAAGA GGCCCCGTTT GCGTGGAGGT TCCGGATAAG 160
TCAACCGAAG TTGACATCTC GGCAAATGTG GGCTACTGAG 200
GCGAAGGCTG ATTCGACATC AGGGGTGTAG TGTATTGCGA 240
*
GGAATAGTCC TGTAAGGATC TGAGAAATAA GGCAAAGGCC 280
AAGTAGTGAG CCAAAGTTT 299;
part of the cytochrome-b gene of YELLOW FIN TUNA having the following sequence: 
AGCCAACGAA   GGCGGTCATC   ATAACTAGGA   GTAGGAGTAC   40
                                        *   .
TACTCCGATG   TTTCATGTTT   CCTTGTATAG   GTAAGAGCCG   80
 
TAGTAAAGTC CTCGGCCGAT GTGGAAGTAG ATGCAGATAA 120
*
AGAAGAAAGA GGCCCCGTTT GCGTGGAGGT TCCGGATAAG 160
TCAACCGAAG TTGACATCTC GGCAAATGTG GGCTACTGAG 200
GCGAAGGCTG ATTCGACATC AGGGGTGTAG TGTATTGCGA 240
GGAATAGTCC TGTAAGGATT TGAGAAATAA GGCAAAGGCC 280
AAGTAGTGAG CCAAAGTTT 299;
part of the part of the cytochrome-b gene of BIG EYE TUNA having the following sequence: 
*
AGCCAACGAA GGCGGTCATC ATAACTAGGA GTAGTAGTAC 40
TACTCCGATG TTTCATGTTT CTTTGTATAG GTAAGAGCCG 80
*
TAGTAAAGTC CTCGGCCGAT GTGGAAGTAG ATACAGATAA 120
AGAAGAAAGA GGCCCCGTTT GCGTGGAGGT TCCGGATGAG 160
TCAACCGAAG TTGACATCTC GGCAAATGTG GGCTACTGAG 200
*
GCGAAGGCTG ATTCGACATC AGGGGTGTAG TGCATTGCGA 240
GGAATAGTCC TGTAAGGATT TGAGAAATAA GGCAAAGGCC 280
AAGTAGTGAG CCAAAGTTT 299;
and part of the cytochrome-b gene of ALBACORE TUNA having the following sequence:

                               *
AGCCAACGAA  GGCGGTCATC  ATAACTAGAA  GTAGGAGTAC      40
                              *
TACTCCGATG  TTTCATGTTT  CTTTGTAGAG  GTAAGAGCCG      80
        *                             *
TAGTAAAGGC  CTCGGCCGAT  GTGGAAGTAG  ATGCAGATAA     120
AGAAGAAAGA  GGCCCCGTTT  GCGTGGAGGT  TCCGGATGAG     160
TCAACCGAAG  TTGACATCTC  GGCAAATGTG  GGCTACTGAG     200
                              *
GCGAAGGCTG  ATTCGACATC  AGGGGTATAG  TGTATTGCGA     240
GGAATAGTCC  TGTAAGGATT  TGAGAAATAA  GGCAAAGGCC     280
AAGTAGTGAG  CCAAAGTTT                              299.

As stated hereinabove, the oligonucleotide primers used in the present invention have been described in Kocher et al. Proc. Natl. Acad. Sci. 86: 6196-6200) (1989).

This oligonucleotide primer is useful in the process of the present invention for many animals, e.g. mammals, birds and fish as has been shown in Table 1 above.

As described above, the laboratory procedures are standard PCR techniques and have been described many times by many people. (This is for the amplification and preparation of double-stranded and single-stranded DNA). The sequencing protocols follow a kit provided by US Biochemical Corporation. The DNA isolation procedure is a modification of previously published methods but the modifications are slight.

The sequences provided herein are part of the non-coding sequence of the cytochrome-b genes of tuna species. This segment of the cytochrome-b protein (inferred from the complementary sequence of that determined) is identical in the four species of *Thunnus* tuna that were examined according to the process of this invention.

### (iv) Brief Description of the Drawings

In the accompanying drawings,
Fig. 1 is a copy of the autoradiograph described in the data analysis;
Fig. 2 is a representation of the nucleotide sequence of an amplified segment; and
Figs. 3A, 3B and 3C, collectively designated Fig. 3, show the intra-specific variation at the cytochrome-b gene locus for each of the species tested and as described in the data analysis.

### (v) Best Mode For Carrying Out Invention

Before describing the examples, some preliminary experiments will be described.

The most common procedure for isolating DNA uses protease digestion in the presence of the anionic detergent SDS followed by organic extraction (phenol and/or chloroform) and then ethanol precipitation of the DNA. This method has been very successful and high molecular weight DNA is usually obtained from fresh tissue. However, many of the specimens that one encounters in forensic work are far from fresh and meat inspectors and game wardens often have to deal with meat that is "well hung" or has been processed.

The preferred procedure for isolating "good quality" DNA from fish tissues which routinely yielded DNA capable of being amplified by PCR was a modification of a method designed to isolate RNA (see Chomczynski, P. and N. Sacchi. 1987. Single step method of RNA isolation by acid quanidinium thiocyanate-phenol-chloroform extraction. Anal. Biochem. 162:156-159.) A modification of this method has been used to prepare DNA for fingerprint analysis. (see B.J. Turner, J.E. Elder and T.F. Laughlin. 1989. DNA fingerprinting of fishes: a general method using oligonucleotide probes. Fingerprint News 4(4):15-16.)

Several factors had to be considered before a specific segment of DNA was selected for amplification. This region of the genome has to accept mutations sufficiently quickly that closely related organisms have different sequences, but not so rapidly that the amount of variation within a species is substantial. The length of segment had to be long enough to permit the detection of sequence differences between congeneric species, but short enough that its nucleotide sequence could be determined from a single loading of a standard DNA sequencing gel. A protein coding region of a gene was preferred because amplification and/or sequencing errors could be detected by translating the nucleotide sequence and comparing it with known amino acid sequences of the gene. A knowledge of the pattern of mutations in the gene would be an asset as would information on the structure and function of the corresponding protein, especially invariant amino acids. This would also facilitate comparing the nucleotide sequence from a sample with those in a data bank. Moreover, the creation of a data bank is a lengthy process and so it was desirable to take advantage of a gene whose sequence had been determined in many diverse organisms. The mitochondrially encoded gene for cytochrome-b satisfies these criteria admirably.

Part of the nucleotide sequence of the gene for cytochrome-b has been determined for many vertebrates including mammals, birds, and fish. Thus, a substantial data base already exists. The amino acid sequence of cytochrome-b is highly conserved but because of the degeneracy of the genetic code, the genes for cytochrome-b differ by at least a few nucleotides even in very closely related species. A mitochondrially encoded gene has the additional advantage of behaving as if it were a haploid locus and therefore the problem of heterozygosity is avoided (heteroplasmy being rare in mitochondria). It is now known that one pair of primers could be used to amplify a 307 basepair segment of the cytochrome-b gene from any vertebrate that was tested. The cytochrome-b gene corresponding to amino acids 33 to 134 is the segment of choice for FINS. Other regions may also be used: e.g. ribosomal RNA genes in bacteria.

The following are examples of the invention:

### EXPERIMENT 1

### FIRST PROCEDURE FOR THE EXTRACTION OF DNA

DNA is extracted from tissue (e.g. muscle, hair, blood or bone) by homogenizing the sample in 4 M guanidinium thiocyanate, 25 mM sodium citrate, pH 7.0, 0.5% sarcosyl and 0.1 M 2-mercaptoethanol. Following homogenization, the solution is extracted with phenol:chloroform:isoamyl alcohol (25:24:1). The phases are separated by centrifugation and to the aqueous phase is added an equal volume of isopropanol. The mixture is left overnight at -20°C and then centrifuged to pellet the DNA. The DNA pellet is washed with cold (-20°C) 70% ethanol, dried under vacuum, dissolved in 10 mM Tris/HC1, 1 mM EDTA, pH 8.0 and stored at 4°C. This crude total (mitochondrial and genomic) DNA extract is used as the substrate for the amplification procedure.

### SECOND PROCEDURE FOR THE EXTRACTION OF DNA

### Source and Identification of Tuna

Samples of skeletal muscle were collected from the dorsal fin area of different tuna species by fisheries officers from Canada's Department of Fisheries and Oceans. The tuna were caught off southwest Nova Scotia. Samples of bluefin tuna from the Virgin Rock off Newfoundland were also examined. These samples were bagged individually, labelled, and either kept on ice or frozen at -20°C before being shipped in locked containers to the Biochemistry Department at Memorial University in St. John's, Newfoundland. Full capture data including otolith number and a photo log of each sample were maintained. In addition, representative specimens of the four species of tuna examined in this study were delivered whole to the laboratory where the experiments were carried out.

### Electrophoretic Analysis of Muscle Proteins

Approximately 0.2 g of muscle tissue was homogenised by hand using a plastic pestle (Mandel Scientific) in a 1.5 mL EPPENDORF_{TM} tube in 1 mL of 12 mM Tris/borate buffer, pH 9.0. The homogenate was centrifuged at 12000 x g for 15 min at 4°C. The supernatant was diluted 10 fold with sterile, deionised water and approximately 0.5 »L was applied to a Pharmacia isoelectric-focusing PHASTGEL_{TM} system (pH range 3-9). After the isoelectric-focusing was completed, the gel was stained with Coomassie brilliant blue R350. Precast isoelectric-focusing gels and protein standards were obtained from Pharmacia (Canada) Ltd., Dorval, Que.

Briefly, 2-5g of muscle was cut into small pieces and homogenised directly at room temperature with a glass-TEFLON_{TM} homogeniser in 10 mL of a solution containing 4 M guanidinium thiocyanate, 25 mM sodium citrate, pH 7.0, 0.5% sarcosyl, and 0.1 M 2-mercaptoethanol. One and a half millilitres of 2 M sodium acetate, pH 4.1, was then added to the homogenate and the solution was mixed with 10 mL of water-saturated phenol and 4 mL of chloroform/isoamyl alcohol (24:1, v/v). After shaking, the mixture was left on ice for 15 min and then centrifuged at 10,000 x g for 20 min at 4°C. The aqueous phase was transferred to a fresh tube containing 10 mL of isopropanol. The solutions were mixed and left at -20°C for at least 1 h. Precipitated nucleic acids were obtained by centrifugation at 10,000 x g for 20 min at 4°C. The pellet was washed with cold (-20°C) 70% ethanol, dried briefly under vacuum, and dissolved in 10 mM Tris/HCl, 1 m M EDTA, pH 8.0, and stored at 4°C. This crude nucleic acid preparation was suitable for DNA amplification.

### THIRD PROCEDURE FOR THE EXTRACTION OF DNA

Ground beef, ground port, lamb chops, chicken breasts, a frozen turkey, partially-cooked battered cod, chicken nuggets, and canned tuna and salmon were purchased from a local supermarket (in the St. John's, Newfoundland, Canada area). Fresh cod fillets, smoked salmon, smoked mackerel, pickled herring, and salt cod were obtained from Fishery Products International (St. John's, Newfoundland). Other biological samples (e.g. blood, tissues not mentioned above, formalin-fixed specimens, skins, etc.) were found in the Faculty of Science, Memorial University, St. John's, Newfoundland.

The sample was cut into small pieces and homogenised directly at room temperature with a glass-TEFLON_{TM} Homogeniser in a solution containing 4 M guanidinium thiocyanate, 25 mM sodium citrate, pH 7.0, 0.5% sarcosyl, and 0.1M 2-mercaptoethanol. For every 10 mL of homogenate, 1.5 mL 2M sodium acetate, pH 4.1 was added and the solution was mixed with 10 mL of Tris-saturated phenol and 4 mL of chloroform/isoamyl alcohol (24:1, v/v). After shaking, the mixture was left on ice for 15 min and then centrifuged at 10,000 x g for 20 min at 4°C. The aqueous phase was transferred to a fresh tube containing 10 mL of isopropanol. The solutions were mixed and left at -20°C for at least one hour. Precipitated nucleic acids were obtained by centrifuging at 10,000 x g for 20 min at 4°C. The pellet was washed with cold 70% ethanol, dried briefly under vacuum, and dissolved in 10 mmM Tris/HCl, 1 mM EDTA, pH 8.0 and stored at 4°C. This crude nucleic acid preparation was suitable for DNA amplification.

### Synthesis of Oligonucleotide Primers

The oligonucleotide primers were synthesised using cyanoethyl phosphoramidite chemistry in the Biochemistry Department, Memorial University, St. John's, Newfoundland on a MILLIGEN/BIOSEARCH_{TM} cyclone DNA synthesizer in the DMT-off mode. It was assumed that all steps in the synthesis and recovery of an oligonucleotide had efficiencies of 100%. It was not necessary to purify the oligonucleotides prior to use. The primers used, cyt bH and cyt bL, were 5'CCCCTCAAATGATATTTGTCCTCA3' and 5'CCATCCAACATCTCAGCATGATGAAA3', respectively.

### EXPERIMENT 2

### AMPLIFICATION OF DEFINED SEGMENTS OF THE DNA

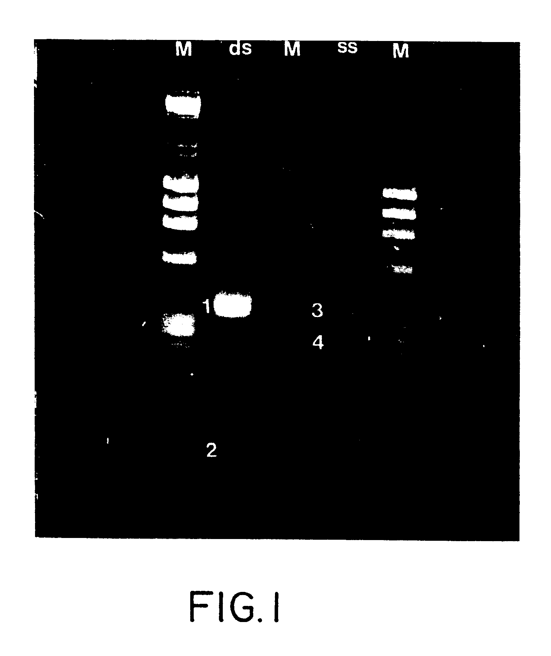

One procedure is as follows:
Two oligonucleotides, that flank the defined segment of the dna to be amplified, are chemically synthesized. These are the primers for the amplification reaction. The amplification reaction is carried out in a final volume of 25 »l in microcentrifuge tubes in a solution containing 67 mm Tris/HCl; pH 8.8: 2 mM magnesium chloride; 10 mM 2-mercaptoethanol; 0.2 mM each of dATP, dCTP, dGTP and dTTP; 10 pmoles of each primer; and 1 unit of a heat-stable, prokaryotic DNA polymerase. Amplification is carried out by 30 - 40 cycles of : 92°C for 45 seconds as the denaturation step, 50°C for 45 seconds as the annealing step, and 72°C for 90 seconds as the extension step. Fifteen »l of the reaction mixture is electrophoresed at 4°C in a 2% low melting agarose gel made with a 40 mm Tris, 30 mM sodium acetate, pH 7.4 Buffer. The DNA is visualized by staining with ethidium bromide and the amplified fragment of DNA is excised from the gel and melted in 100 »L of water at 65°C for five minutes. The sample solution is used to prepare a single-stranded template by asymmetric amplification, except that 40 pmoles of one of the primers is used and only 0.4 pmoles of the second primer is present. Successful amplification after 30 - 40 cycles of 92°C for 45 seconds, 50°C for 45 seconds, and 72°C for 90 seconds is determined by electrophoretic analysis of 5 »L of the reaction mixture in a 2% agarose gel. After staining with ethidium bromide, three bands are visible: a double-stranded band, a middle single-stranded band and a lower faint band containing excess primer (see Figure 1). Residual nucleotides, primers and buffer salts are removed from the single stranded product by centrifugal dialysis. The single-stranded DNA product is obtained in a final volume of approximately 60 »L and 7 »L is used for DNA sequencing.

### Amplification of the Mitochondrial Cytochrome-b Gene

Another procedure for amplifying the mitochondrial cytochrome-b gene from a crude total cellular DNA preparation obtained from tuna muscle is as follows, namely one which was adapted from the method of Kocher et al. (see Kocher, T.D., W.K. Thomas, A. Meyer, S.V. Edwards, S. Paabo, F.X. Villablanca and A.C. Wilson. 1989. Dynamics of mitochondrial DNA evolution in animals: amplification and sequencing with conserved primers. Proc. Natl. Acad. Sci USA 86:6196-6200.) The primers were designated cyt bH and cyt bL. The nucleotide sequence of cyt bH is 5'CCCCTCAGAATGATATTTGTCCTCA3' and that of cyt bL is 5'CCATCCAACATCTCAGCATGATGAAA3'. The primers were synthesized by W.S. Davidson in the Department of Biochemistry at Memorial University, St. John's, Newfoundland on a MILLIGEN/BIOSEARCH_{TM} cyclone DNA synthesizer in the DMT-off mode. It was not necessary to purify the oligonucleotides prior to use. Double-stranded amplifications were carried out in a final volume of 25»l in 0.5 mL microcentrifuge tubes in a reaction cocktail containing 67 mM Tris/HCl, pH 8.8; 2 mM magnesium chloride; 10 mM 2-mercaptoethanol; 0.2 mM each of dATP, dCTP. dGTP, and dTTP; 10 pmol of each primer; and 1 unit of AMPLITAQ_{TM} DNA polymerase (Cetus, Emeryville, CA). A drop of mineral oil was added to cover the reaction mixture and 30 cycles of amplification (92°C for 45 s, 50°C for 45 s, and 72°C for 90 s) were carried out using a PERKIN-ELMER_{TM} (Irvine, CA) DNA THERMAL CYCLER_{TM}. Fifteen microlitres of the reaction mixture was electrophoresed at 4°C through a 2% NUSIEVE_{TM} (FMC BioProducts, Rockland, ME) agarose gel made up and run in a 40 mM Tris, 30 mM sodium acetate buffer titrated to pH 7.4 with HCl. The DNA was visualised by staining with ethidium bromide and the amplified fragment of DNA was excised from the gel and melted in 100 »l of sterile, deionised water. The sample was heated to 65°C for 5 min and mixed vigorously. Five microlitres of this solution was used to prepare a single-stranded template by asymmetric amplification. The reaction was carried out in 100 »L of the same reaction cocktail and under the same cycling conditions as those used for the double-stranded amplification (see above) except that 40 pmol of the cyt bH primer was used and only 0.4 pmol of the cyt bL primer was present. Five microlitres of the reaction mixture was sufficient to visualise double- and single-stranded DNA bands following electrophoresis through a 2% agarose gel and staining with ethidium bromide. Residual nucleotides, primers, and buffer salts were removed from the single-stranded template by centrifugal dialysis using CENTRICON_{TM}-30 filters (Amicon Ltd., Oakville, Ont.). Two millilitres of water was added to the reaction mixture and the solution was applied to the filtration apparatus and centrifuged at 3500 x g for 20 min at 20°C. An additional 2 mL of water was added and the centrifugation repeated. Another 2 mL of water was added and the centrifugation was increased 1 h until the volume in the chamber was approximately 60 »L. Single-stranded DNA template was obtained in this final volume and 7 »L was taken for DNA sequencing.

### Amplification of the Mitochondrial Cytochrome-b Gene

A third procedure for amplifying part of the mitochondrial cytochrome-b gene from a crude nucleic acid extract obtained as described above, was as follows, namely one which was adapted from the method of Kocher et al. (see Kocher, T.D., W.K. Thomas, A. Meyer, S.V. Edwards, S. Paabo, F.X. Villablanca and A.C. Wilson. 1989. Dynamics of mitochondrial DNA evolution in animals: amplification and sequencing with conserved primers. Proc. Natl. Acad. Sci USA 86:6196-6200.)

Double-stranded amplifications were carried out in a final volume of 25 »L in 0.5 mL microcentrifuge tubes in a reaction cocktail containing 67 mM Tris/HCl, pH 8.8; 2 mM magnesium chloride; 10 mM 2-mercaptoethanol; 0.2 mM each of dATP, dCTP, dGTP, and dTTP; 10 pmoles of cyt bH and cyt bL, and 1 unit of AMPLITAQ_{TM} DNA polymerase (Cetus, Emeryville, CA). PROMEGA TAQ_{TM} polymerase from Fisher Scientific, Halifax works equally well using the buffer conditions suggested by the supplier. A drop of mineral oil was added to cover the reaction mixture and 30 cycles of amplification (92°C for 45 s, 50°C for 45 s, and 72°C for 90 s) were carried out using a PERKIN-ELMER (Irvine, CA) DNA THERMAL CYCLER_{TM}. Fifteen »L of the reaction mixture were electrophoresed at 4°C through a 2% NUSIEVE_{TM} (FMC BioProducts, Rockland, ME) agarose gel made up and run in a 40 mM Tris, 30 mM sodium acetate buffer titrated to pH 7.4 with HCl. The DNA was visualised by staining with ethidium bromide and the amplified fragment of DNA was excised from the gel and melted in 100 »L of sterile, deionised water. The sample was heated to 65°C for five minutes and mixed vigorously. Five »L of this solution was used to prepare a single-stranded template by asymmetric amplification. (see Gyllensten, U. and H. Erlich. 1988. Generation of single stranded DNA by the polymerase chain reaction and its application to direct sequencing of the HLA-DQA locus. Proc. Natl. Acad. Sci. USA. 85:7652-7656). The reaction was carried out in 100 »L of the same reaction cocktail and under the same cycling conditions as those used for the double-stranded amplification (see above) except that 40 pmoles of the cyt bH primer was used and only 0.4 pmoles of the cytbL were present. Five »L of the reaction were sufficient to visualise double and single-stranded DNA bands following electrophoresis through a 2% agarose gel and staining with ethidium bromide. Residual nucleotides, primers and buffer salts were removed from the single-stranded template by centrifugal dialysis using CENTRICON_{TM}-30 filters (Amicon Ltd., Oakville, Ont.). Two mL of water were added to the reaction mixture and the solution was applied to the filtration apparatus and centrifuged at 3,500 x g for 20 minutes at 20°C. An additional 2 mL of water were added and the centrifugation repeated. Another 2 mL of water were added and the centrifugation was increased to 1 hour until the volume in the chamber was approximately 60 »L. Single-stranded DNA template was obtained in this final volume and 7 »L were taken for DNA sequencing.

### EXAMPLE I

### SEQUENCING REACTIONS

One procedure for DNA sequencing of 7 »L of the single-stranded amplified product was carried out using a standard dideoxy nucleotide chain termination procedure. Ten pmoles of the limiting primer used in the asymmetric single-stranded amplification reaction is used as the sequencing primer. A typical analysis routinely covers at least 300 nucleotides of the defined segment.

Nucleotide sequencing of the single-stranded template was carried out using Sequenase and a commercial DNA sequencing kit purchased from United States Biochemical Corporation, Cleveland, Ohio. One picomole of cyt bL was used as the primer and α³⁵SdATP was used as the label. The sequencing reactions were performed according to the manufacturer's instructions except that the elongation mix was diluted 50-fold. The sequencing products were separated on 6% polyacrylamide denaturing gels. The primers amplified a 307 base pair (excluding size of primers) segment of the cytochrome-b gene and these procedures routinely allowed the sequence of 290 nucleotides of the non-coding strand to be determined.

A second procedure for nucleotide sequencing of the single-stranded template was carried out using SEQUENASE_{TM} and a commercial DNA sequencing kit purchased from United States Biochemical Corporation, Cleveland, OH. One pmole of cyt bL was used as the sequencing primer and ³⁵SdATP was used as the label. The sequencing reactions were performed according to the manufacturer's instructions except that the elongation mix was diluted fiftyfold. The sequencing products were separated on 6% polyacrylamide denaturing gels. (see Sanger, F., S. Nicklen and R. Coulson. 1977. DNA sequencing with chain-terminating inhibitors. Proc. Natl. Acad. Sci. USA 74:5463-5467.) The primers amplify a 307 base pair (excluding size of primers) segment of the cytochrome-b gene and these procedures routinely allowed the sequence of 290 nucleotides of the non-coding strand to be determined.

### DATA ANALYSIS

The sequence of the defined segment of the DNA of the sample is then compared with the corresponding sequences from known species. If an exact fit is not observed, a cladistic analysis is carried out to predict the closest group of species to the unknown sample. DNA is then extracted from these species and subjected to amplification and sequence analysis. The sequence from the test sample is now compared with these additional sequences, and the procedure is repeated until an identification is made. In many instances it may be important to examine several individuals from different populations of a species in order to gauge the range of variation within that species and to determine if there is population structuring.

### Comparison of Muscle Proteins

Isoelectric-focusing (pH range 3-9) of water-soluble muscle proteins from four tuna species yielded two groups: one characteristic of bluefin and yellowfin tunas and the other of albacore and bigeye. However, it was not possible to discriminate between individual members within either group. Similar results were obtained using a narrower pH range.

### Comparison of Mitochondrial Cytochrome-b Gene Sequences

The nucleotide sequence of a portion of the complementary strand of the mitochondrial cytochrome-b gene was obtained from 33 bluefin, 32 bigeye, 33 yellowfin, and 12 albacore tuna. A consensus sequence of this region was derived by comparing all of the sequences. The numbering system is arbitrary but representative of results that could be obtained with ease from a single loading on a sequencing gel 40 cm in length. Number 296 identifies the nucleotide complementary to the first base of the codon for amino acid 33 (phenylalanine) of tuna cytochrome-b.

As seen in Figures 1, 2 and 3, the nucleotide sequences reveal intraspecific variation in this group of tuna. For example, six genotypes were observed in the 33 bluefin that were examined. Twenty-eight of the bluefin tuna shared an identical genotype. Four of the other five genotypes differed by a single substitution from the consensus for this species, and the fifth had an additional nucleotide change. Four genotypes were observed among the 33 yellowfin examined. The most common genotype was shared by 28 fish. Three other yellowfin genotype was shared by 28 fish. Three other yellowfin had a genotype that differed from the common type at two positions. Two additional yellowfin each had unique genotypes that differed from the common type by a single mutational event. The 12 albacore tuna gave four genotypes. Six albacore shared one genotype, four individuals shared another genotype, and two other genotypes were found in individual fish. These genotypes differ from the most common albacore sequence by one nucleotide substitution. Bigeye tuna showed the most intraspecific variation. Indeed, 11 different genotypes were detected among 32 bigeye tuna tested. There were two main groupings with nine individuals in each. Four bigeye shared a separate genotype as did two pairs. Another six individuals had unique genotypes. The extensive variation within this sample of bigeye suggests that there may be discrete breeding populations of bigeye tuna.

Although intraspecific variation was observed at the cytochrome-b locus in bluefin, bigeye, yellowfin, and albacore tuna, distinctive species-specific sequence differences were also uncovered in each of these four species of tuna. The data are summarised in Table 1.

**Table 1**

| Nucleotides at specific sites can be used to identify tuna species. | | | | | | |
|---|---|---|---|---|---|---|
| | Position | | | | | |
| Species | 35 | 62 | 68 | 89 | 227 | 260 |
| Bluefin | G | T | T | T | G | C |
| Bigeye | T | T | T | T | G | T |
| Yellowfin | G | C | T | T | G | T |
| Albacore | G | T | C/G | G | A | T |

For example, the 32 bigeye tuna that were studied all had a thymine (T) at position 35 whereas members of the three other species had a guanine (G). Similarly, the 33 yellowfin tuna had a cytosine (C) at position 62 whereas a T was present in the other species. Albacore tuna differed from all the other tuna at three sites (68, 89, and 227). The sample of 33 bluefin tuna had a C at position 260 whereas all other tuna examined had a T. These changes appear to be "fixed" in a particular species, thus providing the possibility of using them as genetic markers to define individual members of that species; but this is not the case. For example, the 12 albacore had an adenine (A) at position 29. All of the bluefin and yellowfin tuna had a G at this position as did 29 of 32 bigeye. However, three of the bigeye had an A at position 29. The most likely explanation is that one or more mutations have occurred at this position in the bigeye species. The pattern of nucleotide substitutions in mitochondrial cytochrome-b genes predicts that this will be fairly common. A similar situation is seen at position 158 in bluefin tuna. All but one of the 33 bluefin have an A at this position whereas it is G in all members of the other species. Again, the most likely scenario to explain this observation is that a mutation has occurred at this site in the lineage leading to the variant bluefin. Position 113 provides yet another example of this phenomenon. The consensus sequence at this position was designated R for purine (A or G). Bluefin and bigeye have an A at this site but albacore and 30 of 33 yellowfin tuna had a G. A mutation from G to A in one lineage of yellowfin tuna could account for this change. Rather than using a particular nucleotide at a site to determine the species identify of an individual tuna, it is essential to use all the information available over the entire sequence. This is a form of pattern recognition and calls for a phylogenetic analysis. The nucleotide sequence information provides a frame work for this type of analysis. When the nucleotide sequence of the cytochrome-b gene has been determined for a tuna of unknown species, the sequence can be fitted into the framework by using a computer program, e.g. pylogenetic analysis using parsimony (PAUP). The most likely species identity and an estimate of the reliability of the prediction can thus be calculated. This procedure also takes into account mutations in individual tuna that were not seen in this study.

None of the nucleotide substitutions resulted in a change in amino acid sequence. All but one of the substitutions are third position silent changes; the other detected in one of the bigeye is a first position C to T in a leucine codon and therefore is also silent. Most of the substitutions were transitions (i.e. the interchange of pyrimidines C to T or primers A to G). However four instances of transitions (i.e. a change from a purine to a primidine or vice versa) were noted at positions 32, 35, 68, and 89. This latter type of nucleotide substitution accounted for approximately 20% of the changes observed among the tuna cytochrome-b genes. This pattern of nucleotide substitution has been seen in other comparisons of fish cytochrome-b genes.

### Nucleotide Sequence Analysis

No single nucleotide sequence of the cytochrome-b gene is diagnostic of a particular species. This is because of the nature of mutations in mitochondrial genes coding for proteins and intraspecific variation. This must be taken into account in FINS as it will be impossible to sample enough individuals from all the species that one may wish to have in a data bank to cover all the genetic variation within a species. Therefore, the procedure that is used to overcome this potential problem relies on a phylogenetic analysis. This is a form of pattern recognition and makes use of all the information in a sequence. Fortunately, this field of research is well established in molecular evolutionary studies and computer programs are available (e.g. PAUP). (see Swofford, D.L. 1989. PAUP: phylogenetic analysis using parsimony, Version 3.0b. Illinois Natural History Survey, Champaign, IL.) This procedure also allows reversions and new mutations to be taken into account. Moreover, if the species to which the specimen belongs is not represented in the data base, the phylogenetic analysis will reveal the most closely related species. At this point it would be possible to obtain additional standard species and to expand this section of the data bank. It has been our experience that the amount of intra-specific variation does not preclude the identification of even closely related species. However, there may be some very closely related organisms, that are regarded as separate species based on morphological characteristics, that are not genetically distinguishable at the molecular level. In these instances, it will not be possible to use FINS or any other biochemical genetic method to distinguish members of these species from one another.

### EXAMPLE II

The DNA extracted from samples was examined and it was found that the nucleic acids in the final preparation were usually less than five to seven kilobase pairs in length (estimated by ethidium bromide staining after agarose gel electrophoresis). The protocol outlined above is easily scaled down for small amounts of sample. It is rapid and amplifiable DNA was obtained from a wide variety of samples including processed meats. This is shown below in Table 2.

The FINS procedure was successful in determining the species identify of biological samples that had been processed in the following ways.

**Table 2**

| Process | Samples Tested |
|---|---|
| Freshly frozen | Many mammals, birds, fish |
| Ground meat | Beef, pork |
| Smoked | Salmon, mackerel |
| Pickled | Herring |
| Canned | Salmon, tuna species |
| Salted | Cod |
| Partially cooked | Battered cod, chicken nuggets |
| Formalin fixed | Salmonids |
| Ethanol preservation | Salmonids |
| Blood smears on slides | Birds |
| Preserved skins | Mammals, birds |

The following example illustrates the use of the procedure outlined above.

### EXAMPLE III

Four commercially-important tuna species in the genus *Thunnus* are caught off the east coast of Canada. The harvest of bluefin tuna is regulated, whereas that of bigeye, yellowfin, or albacore is not. However, enforcement of the regulations governing the bluefin tuna fishery was difficult because of the close genetic relationships among these species and the ease with which morphological characters may be removed once a fish has been landed. Furthermore, analysis of muscle proteins using sensitive methods, e.g. isoelectric focusing could not distinguish bluefin tuna from yellowfin tuna.

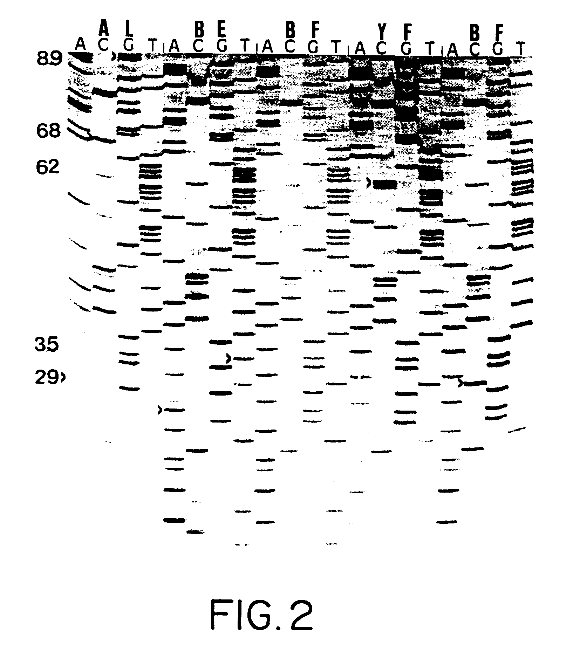

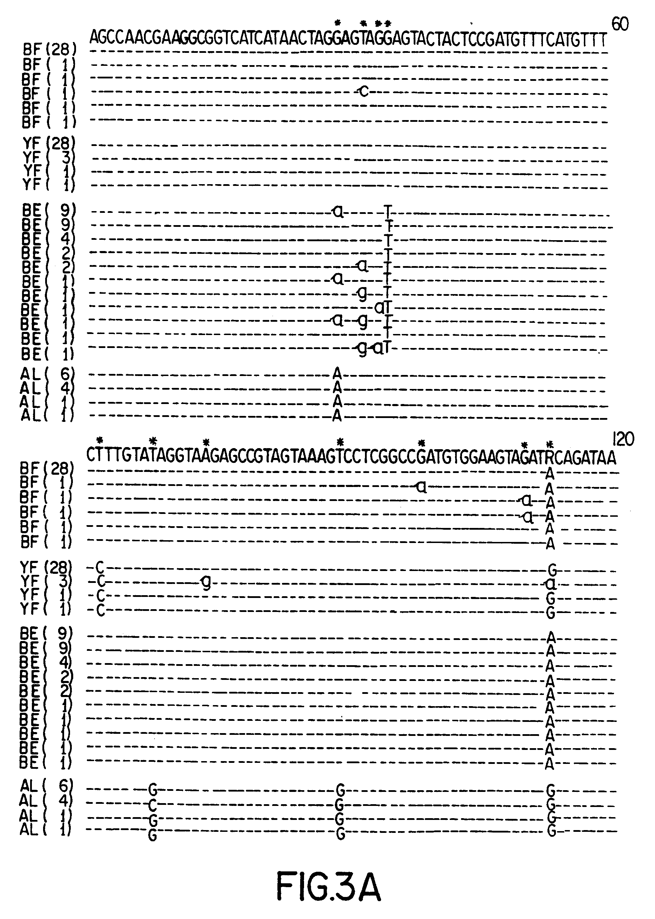

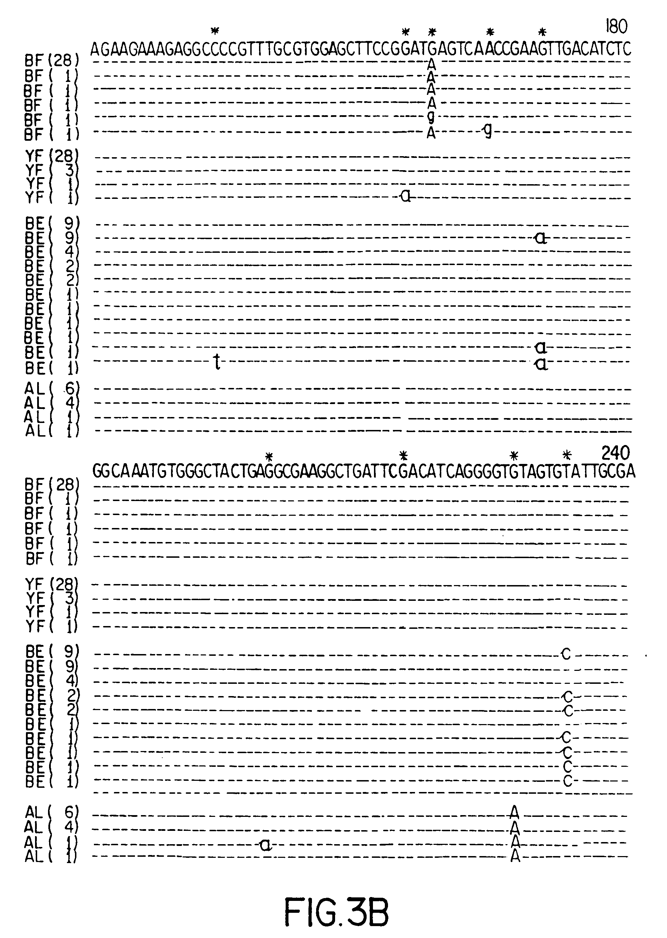

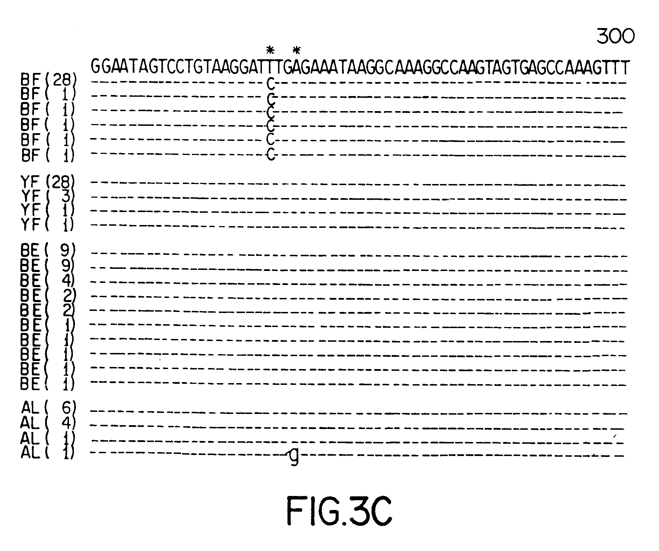

DNA was isolated from muscle samples from commercially caught tuna that had been identified by officers from the Canadian Department of Fisheries and Oceans. The polymerase chain reaction procedure which has been described above and which has also been described in U.S. Patent 4,683,195, the content of which are herein incorporated by reference, was used to amplify a region of the cytochrome-b gene from members of these species. The nucleotide sequence of the amplified segment was determined by the dideoxy chain termination procedure. An example of these results is shown in Figure 2. The corresponding sequences were compared for 33 bluefin, 33 yellowfin, 32 bigeye, and 12 albacore. Intra-specific variation was observed at this locus in each of the species and this is shown in Figure 3. More importantly, there are fixed differences between each of these four species. For example: position 29 in Figure 3 shows that albacore tuna have an A at this position, whereas in the other species of tuna it is a G; position 35 in Figure 3 shows that big eye tuna have a T at this position, whereas in the other species of tuna it is a G; position 62 in Figure 3 shows that yellow fin tuna have a C at this position, whereas in the other species of tuna it is a T; position 68 in Figure 3 shows that albacore tuna have a G at this position, whereas in the other species of tuna it is a T; position 89 in Figure 3 shows that albacore tuna have a G at this position, whereas in the other species of tuna it is a T; position 218 in Figure 3 shows that bluefin tuna have an A at this position, whereas in the other species of tuna it is a G; position 227 in Figure 3 shows that albacore tuna have an A at this position, whereas in the other species of tuna it is a G; and position 260 in Figure 3 shows that bluefin tuna have a C at this position, whereas in the other species of tuna it is a T. This type of species-specific genetic marker can be used to determine unambiguously the species identity of an individual tuna.

Markers have been identified that can readily distinguish between other closely related fish (e.g. Atlantic salmon and brown trout); poultry (e.g. chicken and turkey); domesticated mammals (e.g. cow, pig, sheep, goat, horse); and game (e.g. deer, moose, and caribou).

### (vi) Way In Which The Invention Is Capable of Exploitation in Industry

The advantages and utility of the direct sequence analysis of fragments of DNA amplified via the polymerase chain reaction has been established by the present invention. A very small amount of DNA is required initially and the DNA does not have to be highly purified or of high molecular weight.

The present invention uses a pair of primers that correspond to highly conserved regions of the mitochondrial cytochrome-b genes of vertebrates. These primers, in conjunction with the polymerase chain reaction, were used to amplify a 307 base pair segment of the mitochondrial genome and to determine the sequence of this gene from individual tuna. There are several advantages for using this mitochondrial gene for comparing closely related species. The amino acid sequences of cytochrome-b from several diverse organisms have been determined and a consensus sequence showing invariant amino acid positions has been constructed. This serves as a guide to verify that the nucleotide sequence obtained in a forensic experiment is actually that of a cytochrome-b gene. In addition, the pattern of nucleotide substitutions has been determined for many species of mammals, birds, and fishes. Within a particular species and also between closely related species, transitions are more common than transversions and most substitutions occur at synonymous sites such that the amino acid sequence of the protein is not altered.

The principal advantage of FINS is that FINS is a rapid, accurate and reproducible procedure that is based on established techniques. It is not subject to operator bias and can be performed independently in any lab equipped to carry out standard molecular biology. Thus, an analysis could be confirmed in any other diagnostic lab. FINS produces genetic evidence and this should be acceptable to the legal system. Court cases that have previously been hampered by the lack of genetic evidence can now proceed. For example, FINS allows the discrimination between bluefin tuna (a regulated species) and yellowfin tuna (an unregulated species) whereas this was not achieved by protein electrophoretic procedures. FINS is a rapid, reliable and reproducible procedure that is based on established techniques. Therefore, FINS fills the need for a reliable method for determining the biological identity of a specimen when this is not possible by conventional means.

The keys to identifying different species normally rely heavily on morphological characteristics. However, when an animal has been killed for food or sport, these markers are often destroyed or intentionally removed from the animal. This presents a problem for government agencies who are involved in determining the species origin of an animal or products derived from it in order to enforce conservation and/or health-related regulations. The problem is compounded if the meat of the animal has been processed in any way. The Forensically Informative Nucleotide Sequencing procedure (FINS) overcomes these problems. First, DNA is isolated from a wide range of biological samples including processed foods (e.g. canned, partially cooked, pickled, salted, or smoked) using traditional methods. Second, a specific segment of DNA is amplified using the polymerase chain reaction. Third, the nucleotide sequence of the amplified segment of DNA is determined. Fourth, this nucleotide sequence is subjected to a phylogenetic analysis using a data base and the most closely related species is identified.

The area of the gene which is operative for the method of this invention is the cytochrome-b gene. When used in the method of the present invention the PCR procedure amplifies only is part of the cytochrome-b gene. In the present invention, the non-coding strand of the gene is used to do the analysis in tuna. The protein sequence of the cytochrome-b gene product from tuna is a novel sequence and forms part of the present invention.

The procedure of the present invention examined the DNA sequences of the cytochrome-b genes of bluefin, bigeye, yellowfin, and albacore. The amount of intraspecific variation that was detected in these tuna, particularly bigeye, was quite startling and served to illustrate the resolving power of this procedure.

Although variation is apparent within the *Thunnus* tuna species, there are, more importantly, distinct differences which characterise each of the four species. These species-specific genetic markers make it possible to use this procedure and a phylogenetic analysis to determine unambiguously the species identity of an individual tuna. The methodology described above should prove very useful for enforcing the regulations governing the bluefin tuna fishery off Canada's east coast. The methodology is also readily applicable to other enforcement and forensic problems.

INFORMATION FOR SEQ. I.D. NO. 1
(i) SEQUENCE CHARACTERISTICS:
(A) LENGTH: 299 nucleotides
(B) TYPE: nucleotide
(C) TOPOLOGY: paired linear
(ii) MOLECULE TYPE: DNA
(iii) SEQUENCE DESCRIPTION: SEQ. I.D. NO: 1:
complementary sequence of the part of the cytochrome-b
gene of THUNNUS TUNA

                                 *    * **
AGCCAACGAA  GGCGGTCATC  ATAACTAGGA  GTAGGAGTAC      40
                          *     *       *
TACTCCGATG  TTTCATGTTT  CTTTGTATAG  GTAAGAGCCG      80
         *          *             *    *
TAGTAAAGTC  CTCGGCCGAT  GTGGAAGTAG  ATRCAGATAA     120
                *                        *  *
AGAAGAAAGA  GGCCCCGTTT  GCGTGGAGGT  TCCGGATGAG     160
    *     *                                *
TCAACCGAAG  TTGACATCTC  GGCAAATGTG  GGCTACTGAG     200
                 *             *
GCGAAGGCTG  ATTCGACATC  AGGGGTGTAG  TGTATTGCGA     240

*     *
GGAATAGTCC   TGTAAGGATT   TGAGAAATAA   GGCAAAGGCC   280
AAGTAGTGAG   CCAAAGTTT   299.
INFORMATION FOR SEQ. I.D. NO. 2
(i)   SEQUENCE CHARACTERISTICS:
(A)   LENGTH:   299 nucleotides
(B)   TYPE:   nucleotide
(C)   TOPOLOGY:   paired linear
(ii)   MOLECULE TYPE:   DNA
(iii)   SEQUENCE DESCRIPTION:   SEQ.   I.D.   NO:   2:
complementary sequence of the part of the cytochrome-b
gene of BLUE FIN TUNA
AGCCAACGAA   GGCGGTCATC   ATAACTAGGA   GTAGGAGTAC   40
TACTCCGATG   TTTCATGTTT   CTTTGTATAG   GTAAGAGCCG   80
*
TAGTAAAGTC   CTCGGCCGAT   GTGGAAGTAG   ATACAGATAA   120
*
AGAAGAAAGA   GGCCCCGTTT   GCGTGGAGGT   TCCGGATAAG   160
TCAACCGAAG   TTGACATCTC   GGCAAATGTG   GGCTACTGAG   200
GCGAAGGCTG   ATTCGACATC   AGGGGTGTAG   TGTATTGCGA   240
*
GGAATAGTCC   TGTAAGGATC   TGAGAAATAA   GGCAAAGGCC   280
AAGTAGTGAG   CCAAAGTTT   299.
INFORMATION FOR SEQ. I.D. NO. 3
(i)   SEQUENCE CHARACTERISTICS:
(A)   LENGTH:   299 nucleotides
(B)   TYPE:   nucleotide
(C)   TOPOLOGY:   paired linear
(ii)   MOLECULE TYPE:   DNA
(iii)   SEQUENCE DESCRIPTION:   SEQ.   I.D.   NO:   3:
complementary sequence of the part of the cytochrome-b
gene of YELLOW FIN TUNA
AGCCAACGAA   GGCGGTCATC   ATAACTAGGA   GTAGGAGTAC   40
*
TACTCCGATG   TTTCATGTTT   CCTTGTATAG   GTAAGAGCCG   80
*
TAGTAAAGTC   CTCGGCCGAT   GTGGAAGTAG   ATGCAGATAA   120

*
AGAAGAAAGA   GGCCCCGTTT   GCGTGGAGGT   TCCGGATAAG   160
TCAACCGAAG   TTGACATCTC   GGCAAATGTG   GGCTACTGAG   200
GCGAAGGCTG   ATTCGACATC   AGGGGTGTAG   TGTATTGCGA   240
GGAATAGTCC   TGTAAGGATT   TGAGAAATAA   GGCAAAGGCC   280
AAGTAGTGAG   CCAAAGTTT   299.
INFORMATION FOR SEQ. I.D. NO. 4
(i)   SEQUENCE CHARACTERISTICS:
(A)   LENGTH:   299 nucleotides
(B)   TYPE:   nucleotide
(C)   TOPOLOGY:   paired linear
(ii)   MOLECULE TYPE:   DNA
(iii)   SEQUENCE DESCRIPTION:   SEQ.   I.D.   NO:   4:
complementary sequence of the part of the cytochrome-b
gene of BIG EYE TUNA
*
AGCCAACGAA   GGCGGTCATC   ATAACTAGGA   GTAGTAGTAC   40
TACTCCGATG   TTTCATGTTT   CTTTGTATAG   GTAAGAGCCG   80
*
TAGTAAAGTC   CTCGGCCGAT   GTGGAAGTAG   ATACAGATAA   120
AGAAGAAAGA   GGCCCCGTTT   GCGTGGAGGT   TCCGGATGAG   160
TCAACCGAAG   TTGACATCTC   GGCAAATGTG   GGCTACTGAG   200
*
GCGAAGGCTG   ATTCGACATC   AGGGGTGTAG   TGCATTGCGA   240
GGAATAGTCC   TGTAAGGATT   TGAGAAATAA   GGCAAAGGCC   280
AAGTAGTGAG   CCAAAGTTT   299.
INFORMATION FOR SEQ. I.D. NO. 5
(i)   SEQUENCE CHARACTERISTICS:
(A)   LENGTH:   299 nucleotides
(B)   TYPE:   nucleotide
(C)   TOPOLOGY:   paired linear
(ii)   MOLECULE TYPE:   DNA
(iii)   SEQUENCE DESCRIPTION:   SEQ.   I.D.   NO:   5:
complementary sequence of the part of the cytochrome-b
gene of ALBACORE TUNA
*
AGCCAACGAA   GGCGGTCATC   ATAACTAGAA   GTAGGAGTAC   40

*

TACTCCGATG   TTTCATGTTT   CTTTGTAGAG   GTAAGAGCCG   80

*   *

TAGTAAAGGC   CTCGGCCGAT   GTGGAAGTAG   ATGCAGATAA   120

AGAAGAAAGA   GGCCCCGTTT   GCGTGGAGGT   TCCGGATGAG   160

TCAACCGAAG   TTGACATCTC   GGCAAATGTG   GGCTACTGAG   200

*

GCGAAGGCTG   ATTCGACATC   AGGGGTATAG   TGTATTGCGA   240

GGAATAGTCC   TGTAAGGATT   TGAGAAATAA   GGCAAAGGCC   280

AAGTAGTGAG   CCAAAGTTT   299.

## Claims

1. A method for determination of the genus, and then the species, strain, sub-species or sub-set of sample of a eukaryote or a prokaryote organism, or for the determination of the species origin of a sample of an organism or the population identity of said organism, characterized by the steps of:
- isolating DNA from said sample;
- amplifying a defined segment of said DNA;
- first determining if there is a match of the DNA sequence of said sample with any DNA sequence in a data base of DNA sequences from known species by comparing that DNA sequence with said data base to establish the identity of said sample;
- then if no direct match is observed, carrying out a cladistic analysis to determine the closest species by the additional steps of: extracting DNA from a close genus, species, strain, sub-species or sub-set; amplifying and sequencing segments of interest therefrom; and comparing that sequenced segment with the sequence from the sample; and repeating said above-defined additional steps until a match is found and the identity of the sample established.

2. The method of claim 2 characterized in that said organism is a mammal, a bird, a reptile, an amphibian, a fish or an invertebrate.

3. The method of claim 1 characterized in that the amplification of said DNA is carried out using primer cyt bH 5'CCCCTCAAATGATATTTGTCCTCA3' and primer cyt bL 5'CCATCCAACATCTCAGCATGATGAAA3', respectively.

4. The method of claim 2 characterized in that said method is carried out to distinguish mammals, birds, and fish in freshly frozen samples; or to distinguish between closely related fish, namely Atlantic salmon and brown trout; or to distinguish between domesticated animals, namely cow, pig, sheep, goat and horses; or to distinguish between poultry, namely chicken and turkey; or to distinguish between game, namely deer, moose and caribou; or to distinguish between salmon and mackerel in smoked samples; or to distinguish between herring species in pickled samples; or to distinguish between salmon and tuna species in canned samples; or to distinguish between cod species in salted samples or to distinguish between battered cod and chicken nuggets in partially cooked samples; or to distinguish between various birds in blood smears in slide samples; or to distinguish between various mammals and birds in preserved skin samples.

5. The method of claim 3 characterized in that said method is carried out to distinguish the following species of tuna by the presence of nucleotide at specific sites according to the following table:
| | Position | | | | | |
|---|---|---|---|---|---|---|
| Species | 35 | 62 | 68 | 89 | 227 | 260 |
| Bluefin | G | T | T | T | G | C |
| Bigeye | T | T | T | T | G | T |
| Yellowfin | G | C | T | T | G | T |
| Albacore | G | T | C/G | G | A | T |

6. The method of claim 1 characterized in that said DNA is isolated from said sample by homogenizing tissue from which DNA is to be isolated; extracting the homogenate; separating the phases by centrifugation; precipitating the DNA from the aqueous phase with an alcohol; collecting the DNA as a pellet; and using the crude total mitochondrial and genomic DNA in the pellet.

7. The method of claim 6 characterized in that said homogenizing step is carried out in an aqueous solution containing guanidinium thiocyanate, sodium citrate, sarcosyl and mercaptoethanol.

8. The method of claim 6 characterized in that said extraction step is carried out with a mixture of phenol: chloroform:isoamyl alcohol.

9. The method of claim 6 characterized in that the collecting of said DNA is carried out by separating the phases by centrifugation, adding an equal volume of isopropanol to the aqueous phase, cooling to -20°C and centrifuging to pellet the DNA.

10. The method of claim 6 characterized in that the DNA is obtained by the steps of washing the pellet with cold ethanol, drying the DNA pellet, and dissolving the dried pellet in a solution of Tris/HC1 and EDTA.

11. The method of claim 1 characterized in that crude mitochondrial or genomic DNA is amplified by first chemically synthesizing two oligonucleotides that flank the defined segment of the genome to be amplified; using these oligonucleotides as primers in a series of cycles which include separating double-stranded DNA, annealing the primers to the template DNA, and elongating the defined segment of DNA using a heat-stable DNA polymerase and deoxynucleotide triphosphate precursors; electrophoresing the reaction mixture, staining the DNA and excising the amplified segment of DNA from the gel; preparing a single-stranded template by asymmetric amplification; removing residual nucleotides, primers and buffer salts from the single-stranded DNA by centrifugal dialysis; and recovering the single-stranded DNA.

12. The method of claim 10 characterized in that said amplification reaction is carried out in an aqueous solution containing Tris/HCl, magnesium chloride, 2-mercaptoethanol, dATP, dCTP, dGTP, dTTP, each primer, and a heat-stable, prokaryotic DNA polymerase.

13. The method of claim 10 characterized in that said amplification is carried out by about 30 - about 40 cycles of: about 92°C for about 45 seconds as the denaturation step, about 50°C for about 45 seconds as the annealing step, and about 72°C for about 90 seconds as the extension step.

14. The method of claim 10 characterized in that said excising of said DNA is carried out by first electrophoresing the reaction mixture in a low melting agarose gel made with a Tris, sodium acetate, buffer; visualising the DNA by staining with ethidium bromide and excising the amplified segment of DNA from the gel and melting in water.

15. The method of claim 10 characterized in that the single-stranded template is prepared by asymmetric amplification in an aqueous solution containing Tris/HCl, magnesium chloride, 2-mercaptoethanol, dATP, dCTP, dGTP, dTTP, each primer, and a heat-stable, prokaryotic DNA polymerase.

16. The method of claim 1 characterized in that DNA sequencing of the single-stranded amplified product is carried out using the dideoxy chain termination DNA sequencing procedure.

17. The method of claim 15 characterized in that the limiting primer used in the asymmetric single-stranded amplification reacting is used as the sequencing primer.

18. The method of claim 15 characterized in that the analysis routinely covers at least 299 nucleotides of the defined segment.

19. Complementary sequence of the part of the cytochrome-b gene of THUNNUS TUNA having the following sequence:

                                   *    *  **
AGCCAACGAA  GGCGGTCATC  ATAACTAGGA  GTAGGAGTAC   40
                            *     *       *
TACTCCGATG  TTTCATGTTT  CTTTGTATAG  GTAAGAGCCG   80
          *          *            *    *
TAGTAAAGTC  CTCGGCCGAT  GTGGAAGTAG  ATRCAGATAA  120
                 *                         *  *
AGAAGAAAGA  GGCCCCGTTT  GCGTGGAGGT  TCCGGATGAG  160
     *     *                                 *
TCAACCGAAG  TTGACATCTC  GGCAAATGTG  GGCTACTGAG  200
                  *             *
GCGAAGGCTG  ATTCGACATC  AGGGGTGTAG  TGTATTGCGA  240
                       *   *
GGAATAGTCC  TGTAAGGATT  TGAGAAATAA  GGCAAAGGCC  280
AAGTAGTGAG  CCAAAGTTT                           299.

20. Complementary sequence as claimed in claim 18 comprising the part of the cytochrome-b gene of BLUE FIN TUNA having the following sequence:

AGCCAACGAA GGCGGTCATC ATAACTAGGA GTAGGAGTAC 40
TACTCCGATG TTTCATGTTT CTTTGTATAG GTAAGAGCCG 80

*
TAGTAAAGTC CTCGGCCGAT GTGGAAGTAG ATACAGATAA 120
*
AGAAGAAAGA GGCCCCGTTT GCGTGGAGGT TCCGGATAAG 160
TCAACCGAAG TTGACATCTC GGCAAATGTG GGCTACTGAG 200
GCGAAGGCTG ATTCGACATC AGGGGTGTAG TGTATTGCGA 240
*
GGAATAGTCC TGTAAGGATC TGAGAAATAA GGCAAAGGCC 280
AAGTAGTGAG CCAAAGTTT 299.

21. Complementary sequence as claimed in claim 18 comprising of the part of the cytochrome-b gene of YELLOW FIN TUNA having the following sequence:

AGCCAACGAA GGCGGTCATC ATAACTAGGA GTAGGAGTAC 40
*
TACTCCGATG TTTCATGTTT CCTTGTATAG GTAAGAGCCG 80
*
TAGTAAAGTC CTCGGCCGAT GTGGAAGTAG ATGCAGATAA 120
*
AGAAGAAAGA GGCCCCGTTT GCGTGGAGGT TCCGGATAAG 160
TCAACCGAAG TTGACATCTC GGCAAATGTG GGCTACTGAG 200
GCGAAGGCTG ATTCGACATC AGGGGTGTAG TGTATTGCGA 240
GGAATAGTCC TGTAAGGATT TGAGAAATAA GGCAAAGGCC 280
AAGTAGTGAG CCAAAGTTT 299.

22. Complementary sequence as claimed in claim 18 comprising the part of the cytochrome-b gene of BIG EYE TUNA having the following sequence:

                                                *
AGCCAACGAA  GGCGGTCATC  ATAACTAGGA  GTAGTAGTAC    40
TACTCCGATG  TTTCATGTTT  CTTTGTATAG  GTAAGAGCCG    80
                                              *
TAGTAAAGTC  CTCGGCCGAT  GTGGAAGTAG  ATACAGATAA   120
AGAAGAAAGA  GGCCCCGTTT  GCGTGGAGGT  TCCGGATGAG   160

TCAACCGAAG TTGACATCTC GGCAAATGTG GGCTACTGAG 200
*
GCGAAGGCTG ATTCGACATC AGGGGTGTAG TGCATTGCGA 240

GGAATAGTCC TGTAAGGATT TGAGAAATAA GGCAAAGGCC 280
AAGTAGTGAG CCAAAGTTT 299.

23. Complementary sequence as claimed in claim 18 comprising the part of the cytochrome-b gene of ALBACORE TUNA having the following sequence:

                              *
AGCCAACGAA GGCGGTCATC ATAACTAGAA GTAGGAGTAC   40
                             *
TACTCCGATG TTTCATGTTT CTTTGTAGAG GTAAGAGCCG   80
     *                              *
TAGTAAAGGC CTCGGCCGAT GTGGAAGTAG ATGCAGATAA  120
AGAAGAAAGA GGCCCCGTTT GCGTGGAGGT TCCGGATGAG  160
TCAACCGAAG TTGACATCTC GGCAAATGTG GGCTACTGAG  200
                            *
GCGAAGGCTG ATTCGACATC AGGGGTATAG TGTATTGCGA  240
GGAATAGTCC TGTAAGGATT TGAGAAATAA GGCAAAGGCC  280
AAGTAGTGAG CCAAAGTTT                         299.

24. A kit comprising a carrier; compartments within said carrier to receive one container containing DNA from a defined eukaryote or prokaryote organism; a second container containing an amplified segment of the DNA from said defined organism; and a third container containing DNA sequences for at least one of a plurality of genera, species, strain, sub-species, sub-set, and DNA derived therefrom.

25. The kit of claim 24 wherein the organism is a mammal, a bird, a reptile, an amphibian, a fish or an invertebrate.

26. The kit of claim 24 comprising a carrier; compartments within said carrier to receive one container containing DNA from a defined organism; a second container containing an amplified segment of the DNA from the defined organism; and a third container containing DNA sequences for a plurality of species or DNA derived therefrom.

## Patentansprüche

1. Verfahren zur Bestimmung der Gattung und dann der Art, des Stammes, der Unterart oder Unterordnung einer Probe eines eukaryotischen oder eines prokaryotischen Organismus oder zur Bestimmung des Ursprungs der Art einer Probe eines Organismus oder der Identität der Population des Organismus, **gekennzeichnet** durch die Stufen:
- Isolierung der DNA aus der Probe,
- Amplifikation eines definierten Segments der DNA,
- erste Bestimmung, ob die DNA-Sequenz der Probe mit irgendeiner DNA-Sequenz in einer Datenbank von DNA-Sequenzen aus bekannten Arten übereinstimmt, indem die DNA-Sequenzen mit der Datenbank verglichen werden, um die Identität der Probe zu ermitteln,
- anschließend, wenn keine direkte Übereinstimmung festgestellt wird, Durchführen einer cladistischen Analyse, um die nächste Art durch die zusätzlichen Stufen: Extrahieren der DNA aus einer nahen Gattung, Art, Stamm, Unterart oder Unterordnung, Amplifizieren und Sequenzieren der Segmente daraus, die von Interesse sind, und Vergleichen des sequenzierten Segments mit der Sequenz aus der Probe, zu bestimmen, und Wiederholen der vorstehend definierten zusätzlichen Stufen, bis eine Übereinstimmung gefunden wird und die Identität der Probe festgestellt wird.

2. Verfahren nach Anspruch 1, dadurch **gekennzeichnet**, daß der Organismus ein Säugetier, ein Vogel, ein Reptil, eine Amphibie, ein Fisch oder ein Avertebrat ist.

3. Verfahren nach Anspruch 1, dadurch **gekennzeichnet**, daß die Amplifikation der DNA unter Verwendung der Primers cyt bH 5'CCCCTCAAATGATATTTGTCCTCA3' bzw. des Primers cyt bL 5'CCATCCAACATCTCAGCATGATGAAA3' durchgeführt wird.

4. Verfahren nach Anspruch 2, dadurch **gekennzeichnet**, daß das Verfahren durchgeführt wird, um Säugetiere, Vögel und Fische in frischgefrorenen Proben zu unterscheiden, oder um zwischen nahe verwandten Fischen, d.h. dem Antlantiklachs und der braunen Forelle (brown trout), zu unterscheiden, oder um zwischen Haustieren, d.h. Kuh, Schwein, Schaf, Ziege und Pferden, zu unterscheiden, oder um zwischen Geflügel, d.h. Hühner und Truthahn, zu unterscheiden, oder um zwischen Wild, d.h. Hirsch, Elch und Karibu, zu unterscheiden, oder um zwischen Lachs und Makrele in geräucherten Proben zu unterscheiden, oder um zwischen Heringarten in eingelegten Proben zu unterscheiden, oder um zwischen Lachs und Thunfischarten in Dosenproben zu unterscheiden, oder um zwischen Kabeljauarten in salzhaltigen Proben zu unterscheiden, oder um zwischen bearbeitetem Kabeljau und Hühnerteilen in teilgekochten Proben zu unterscheiden, oder um zwischen den verschiedenen Vögeln in Blutausstrichen in Objektträgerpräparaten zu unterscheiden, oder um zwischen den verschiedenen Säugetieren und Vögeln in konservierten Hautproben zu unterscheiden.

5. Verfahren nach Anspruch 3, dadurch **gekennzeichnet**, daß das Verfahren durchgeführt wird, um die folgenden Thunfischarten durch das Vorhandensein eines Nukleotids an speziellen Positionen gemäß der folgenden Tabelle zu unterscheiden:
| | Position | | | | | |
|---|---|---|---|---|---|---|
| Art | 35 | 62 | 68 | 89 | 227 | 260 |
| Bluefin | G | T | T | T | G | C |
| Bigeye | T | T | T | T | G | T |
| Yellowfin | G | C | T | T | G | T |
| Albacore | G | T | C/G | G | A | T |

6. Verfahren nach Anspruch 1, dadurch **gekennzeichnet**, daß die DNA aus der Probe durch Homogenisieren des Gewebes, aus dem die DNA isoliert werden soll, Extrahieren des Homogenats, Trennen der Phasen durch Zentrifugation, Präzipitieren der DNA aus der wässrigen Phase mit einem Alkohol, Isolieren der DNA als ein Pellet und Verwendung der rohen gesamten mitochondrialen und genomischen DNA in dem Pellet, isoliert wird.

7. Verfahren nach Anspruch 6, dadurch **gekennzeichnet**, daß die Homogenisierungsstufe in einer wässrigen Lösung, enthaltend Guanidiniumthiocyanat, Natriumcitrat, Sarcosyl und Mercaptoethanol, durchgeführt wird.

8. Verfahren nach Anspruch 6, dadurch **gekennzeichnet**, daß die Extraktionsstufe mit einem Gemisch aus Phenol:Chloroform:Isoamylalkohol durchgeführt wird.

9. Verfahren nach Anspruch 6, dadurch **gekennzeichnet**, daß die Isolierung der DNA durch Trennen der Phasen mittels Zentrifugation, Zugabe eines gleichen Volumens an Isopropanol zu der wässrigen Phase, Kühlen auf -20°C und Zentrifugieren, um die DNA zu pelletieren, durchgeführt wird.

10. Verfahren nach Anspruch 6, dadurch **gekennzeichnet**, daß die DNA durch die Stufen Waschen des Pellets mit kaltem Ethanol, Trocknen des DNA-Pellets und Auflösen des getrockneten Pellets in einer Lösung aus Tris/HCl und EDTA erhalten wird.

11. Verfahren nach Anspruch 1, dadurch **gekennzeichnet**, daß die rohe mitchondriale oder genomische DNA dadurch amplifiziert wird, daß zuerst chemisch zwei Oligonucleotide synthetisiert werden, die die definierte Sequenz des Genoms, die amplifiziert werden soll, flankieren; diese Oligonucleotide als Primer in einer Reihe von Zyklen verwendet werden, die die Trennung der doppelsträngigen DNA, die Assoziierung der Primer an die Matrizen-DNA und die Verlängerung des definierten DNA-Segments unter Verwendung einer hitzestabilen DNA-Polymerase und Desoxynucleotidtriphosphatvorläufern, elektrophoretische Untersuchung des Reaktionsgemisches, Anfärben der DNA und Herausschneiden des amplifizierten DNA-Segments aus dem Gel umfassen, eine einzelsträngige Matrize durch asymmetrische Amplifikation hergestellt wird, die restlichen Nucleotide, die Primer und Puffersalze aus der einzelsträngigen DNA durch Dialyse in einer Zentrifuge entfernt werden und die einzelsträngige DNA isoliert wird.

12. Verfahren nach Anspruch 10, dadurch **gekennzeichnet**, daß die Amplifikationsreaktion in einer wässrigen Lösung, die Tris/HCl, Magnesiumchlorid, 2-Mercaptoethanol, dATP, dCTP, dGTP, dTTP, jeden Primer und eine hitzestabile prokaryotische DNA-Polymerase enthält, durchgeführt wird.

13. Verfahren nach Anspruch 10, dadurch **gekennzeichnet**, daß die Amplifikation über etwa 30 bis 40 Zyklen aus etwa 92°C für etwa 45 Sekunden als Denaturierungsstufe, etwa 50°C für etwa 45 Sekunden als Assoziierungsstufe und etwa 72°C für etwa 90 Sekunden als Verlängerungsstufe durchgeführt wird.

14. Verfahren nach Anspruch 10, dadurch **gekennzeichnet**, daß das Herausschneiden der DNA durch eine erste elektrophoretische Untersuchung des Reaktionsgemisches in einem niedrigschmelzenden Agarosegel, das mit einem Tris-Natriumacetatpuffer hergestellt wurde, Sichtbarmachen der DNA durch Anfärben mit Ethidiumbromid und Herausschneiden des amplifizierten DNA-Segments aus dem Gel und Schmelzen in Wasser durchgeführt wird.

15. Verfahren nach Anspruch 10, dadurch **gekennzeichnet**, daß die einzelsträngige Matrize durch asymmetrische Amplifikation in einer wässrigen Lösung, die Tris/HCl, Magnesiumchlorid, 2-Mercaptoethanol, dATP, dCTP, dGTP, dTTP, jeden Primer und eine hitzestabile prokaryotische DNA-Polymerase enthält, durchgeführt wird.

16. Verfahren nach Anspruch 1, dadurch **gekennzeichnet**, daß die Sequenzierung der DNA des einzelsträngigen amplifizierten Produkts unter Verwendung des Didesoxykettenabbruch-DNA-Sequenzierverfahrens durchgeführt wird.

17. Verfahren nach Anspruch 16, dadurch **gekennzeichnet**, daß der begrenzende Primer, der bei der asymmetrischen einzelsträngigen Amplifikationsreaktion verwendet wird, als Sequenzierprimer verwendet wird.

18. Verfahren nach Anspruch 15, dadurch **gekennzeichnet**, daß die Analyse routinemäßig mindestens 299 Nucleotide des definierten Segments umfaßt.

19. Komplementäre Sequenz des Teils des Cytochrom-b Gens von THUNNUS TUNA mit der folgenden Sequenz

                              *       *  **
AGCCAACGAA GGCGGTCATC ATAACTAGGA GTAGGAGTAC   40
                         *        *           *
TACTCCGATG TTTCATGTTT CTTTGTATAG GTAAGAGCCG   80
          *                *                    *       *
TAGTAAAGTC CTCGGCCGAT GTGGAAGTAG ATRCAGATAA  120
                    *                                  *    *
AGAAGAAAGA GGCCCCGTTT GCGTGGAGGT TCCGGATGAG  160
     *        *                                            *
TCAACCGAAG TTGACATCTC GGCAAATGTG GGCTACTGAG  200
                      *                     *
GCGAAGGCTG ATTCGACATC AGGGGTGTAG TGTATTGCGA  240
                              *     *
GGAATAGTCC TGTAAGGATT TGAGAAATAA GGCAAAGGCC  280
AAGTAGTGAG CCAAAGTTT                         299

20. Komplementäre Sequenz nach Anspruch 18, umfassend den Teil des Cytochrom-b Gens von BLUE FIN TUNA mit der folgenden Sequenz:

AGCCAACGAA GGCGGTCATC ATAACTAGGA GTAGGAGTAC 40
TACTCCGATG TTTCATGTTT CTTTGTATAG GTAAGAGCCG 80
*
TAGTAAAGTC CTCGGCCGAT GTGGAAGTAG ATACAGATAA 120
*
AGAAGAAAGA GGCCCCGTTT GCGTGGAGGT TCCGGATAAG 160
TCAACCGAAG TTGACATCTC GGCAAATGTG GGCTACTGAG 200
GCGAAGGCTG ATTCGACATC AGGGGTGTAG TGTATTGCGA 240
*
GGAATAGTCC TGTAAGGATC TGAGAAATAA GGCAAAGGCC 280
AAGTAGTGAG CCAAAGTTT 299.

21. Komplementäre Sequenz nach Anspruch 18, umfassend den Teil des Cytochrom-b Gens von YELLOW FIN TUNA mit der folgenden Sequenz:

AGCCAACGAA GGCGGTCATC ATAACTAGGA GTAGGAGTAC 40
*
TACTCCGATG TTTCATGTTT CCTTGTATAG GTAAGAGCCG 80
*
TAGTAAAGTC CTCGGCCGAT GTGGAAGTAG ATGCAGATAA 120
*
AGAAGAAAGA GGCCCCGTTT GCGTGGAGGT TCCGGATAAG 160
TCAACCGAAG TTGACATCTC GGCAAATGTG GGCTACTGAG 200
GCGAAGGCTG ATTCGACATC AGGGGTGTAG TGTATTGCGA 240
GGAATAGTCC TGTAAGGATT TGAGAAATAA GGCAAAGGCC 280
AAGTAGTGAG CCAAAGTTT 299.

22. Komplementäre Sequenz nach Anspruch 18, umfassend den Teil des Cytochrom-b Gens von BIG EYE TUNA mit der folgenden Sequenz:

*
AGCCAACGAA GGCGGTCATC ATAACTAGGA GTAGTAGTAC 40
TACTCCGATG TTTCATGTTT CTTTGTATAG GTAAGAGCCG 80
*
TAGTAAAGTC CTCGGCCGAT GTGGAAGTAG ATACAGATAA 120
AGAAGAAAGA GGCCCCGTTT GCGTGGAGGT TCCGGATGAG 160
TCAACCGAAG TTGACATCTC GGCAAATGTG GGCTACTGAG 200
*
GCGAAGGCTG ATTCGACATC AGGGGTGTAG TGCATTGCGA 240
GGAATAGTCC TGTAAGGATT TGAGAAATAA GGCAAAGGCC 280
AAGTAGTGAG CCAAAGTTT 299.

23. Komplementäre Sequenz nach Anspruch 18, umfassend den Teil des Cytochrom-b Gens von ALBACORE TUNA mit der folgenden Sequenz:

                                   *
AGCCAACGAA  GGCGGTCATC  ATAACTAGAA  GTAGGAGTAC   40
                                  *
TACTCCGATG  TTTCATGTTT  CTTTGTAGAG  GTAAGAGCCG   80
         *                                *
TAGTAAAGGC  CTCGGCCGAT  GTGGAAGTAG  ATGCAGATAA  120
AGAAGAAAGA  GGCCCCGTTT  GCGTGGAGGT  TCCGGATGAG  160
TCAACCGAAG  TTGACATCTC  GGCAAATGTG  GGCTACTGAG  200
                                 *
GCGAAGGCTG  ATTCGACATC  AGGGGTATAG  TGTATTGCGA  240
GGAATAGTCC  TGTAAGGATT  TGAGAAATAA  GGCAAAGGCC  280
AAGTAGTGAG  CCAAAGTTT                           299.

24. Kit, umfassend einen Träger, Kompartmente in dem Träger, um ein Behältnis, enthaltend DNA aus einem definierten eukaryotischen oder prokaryotischen Organismus, ein zweites Behältnis, enthaltend ein amplifiziertes DNA-Segment aus dem definierten Organismus und ein drittes Behältnis, enthaltend DNA-Sequenzen für mindestens eine DNA einer Vielzahl von Gattungen, Arten, Stämmen, Unterarten, Unterordnungen und eine davon abgeleitete DNA, aufzunehmen.

25. Kit nach Anspruch 24, dadurch **gekennzeichnet**, daß der Organismus ein Säugetier, ein Vogel, ein Reptil, eine Amphibie, ein Fisch oder ein Avertebrat ist.

26. Kit nach Anspruch 24, umfassend einen Träger, Kompartmente in dem Träger, um ein Behältnis, enthaltend DNA aus einem definierten Organismus, ein zweites Behältnis, enthaltend ein amplifiziertes Segment der DNA aus dem definierten Organismus, und ein drittes Behältnis, enthaltend DNA-Sequenzen für eine Vielzahl von Arten oder eine davon abgeleitete DNA, aufzunehmen.

## Revendications

1. Méthode pour la détermination du genre et ensuite de l'espèce, de la souche, de la sous-espèce ou du sous-ensemble d'un organisme eucaryote ou procaryote, ou pour la détermination de l'origine d'une espèce d'un échantillon d'un organisme ou de l'identité d'une population de cet organisme, caractérisée par les étapes de :
- isolation de l'ADN de cet échantillon;
- amplification d'un segment défini de cet ADN,
- détermination préalable s'il y a une correspondance de la séquence d'ADN de cet échantillon avec une quelconque séquence d'ADN dans une base de données de séquences d'ADN provenant d'espèces connues par comparaison de cette séquence d'ADN avec cette base de données pour établir l'identité de cet échantillon;
- ensuite, si aucune correspondance directe n'est observée, réalisation d'une analyse cladistique pour déterminer l'espèce la plus proche par les étapes supplémentaires : d'extraction de l'ADN d'un genre, d'une espèce, d'une souche, d'une sous-espèce ou d'un sous-ensemble proches; d'amplification et de séquençage de segments intéressants de celui-ci; et de comparaison de ce segment séquencé avec la séquence provenant de l'échantillon; et de répétition de ces étapes supplémentaires définies plus haut jusqu'à ce qu'une correspondance soit trouvée et que l'identité de l'échantillon soit établie.

2. Méthode suivant la revendication 1, caractérisée en ce que cet organisme est un mammifère, un oiseau, un reptile, un amphibien, un poisson ou un invertébré.

3. Méthode suivant la revendication 1, caractérisée en ce que l'amplification de cet ADN est effectuée avec l'amorce cyt bH 5'-CCCCTCAAATGATATTTGTCCTCA-3' et l'amorce cyt bL 5'-CCATCCAACATCTCAGCATGATGAAA-3', respectivement.

4. Méthode suivant la revendication 2, caractérisée en ce qu'elle est effectuée pour établir une distinction entre des mammifères, des oiseaux et des poissons dans des échantillons fraîchement gelés; ou pour établir une distinction entre des poissons étroitement apparentés, à savoir le saumon de l'Atlantique et la truite brune; ou pour établir une distinction entre des animaux domestiques, à savoir la vache, le porc, le mouton, la chèvre et les chevaux; ou pour établir une distinction entre des volailles, à savoir le poulet et la dinde; ou pour établir une distinction entre des gibiers, à savoir le cerf, l'élan et le caribou; ou pour établir une distinction entre le saumon et le maquereau dans des échantillons fumés; ou pour établir une distinction entre des espèces de harengs dans des échantillons saumurés; ou pour établir une distinction entre le saumon et une espèce de thon dans des échantillons conservés en boîte; ou pour établir une distinction entre des espèces de morue dans des échantillons salés ou pour établir une distinction entre des beignets de morue et des croquettes de poulet dans des échantillons partiellement cuits; ou pour établir une distinction entre divers oiseaux dans des frottis de sang dans des échantillons sur lamelle; ou pour établir une distinction entre divers mammifères et oiseaux dans des échantillons de peau conservés.

5. Méthode suivant la revendication 3, caractérisée en ce qu'elle est effectuée pour établir une distinction entre les espèces suivantes de thon par la présence de nucléotide à des sites spécifiques suivant le tableau suivant :
| | Position | | | | | |
|---|---|---|---|---|---|---|
| Espèce | 35 | 62 | 68 | 89 | 227 | 260 |
| Bluefin | G | T | T | T | G | C |
| Bigeye | T | T | T | T | G | T |
| Yellowfin | G | C | T | T | G | T |
| Albacore | G | T | C/G | G | A | T |

6. Méthode suivant la revendication 1, caractérisée en ce que cet ADN est isolé à partir de cet échantillon par homogénéisation d'un tissu à partir duquel l'ADN doit être isolé; extraction de l'homogénat; séparation des phases par centrifugation; précipitation de l'ADN de la phase aqueuse avec un alcool; récupération de l'ADN sous forme d' un culot; et utilisation de l'ADN total brut mitochondrial et génomique dans le culot .

7. Méthode suivant la revendication 6, caractérisée en ce que cette étape d'homogénéisation est conduite dans une solution aqueuse contenant du thiocyanate de guanidinium, du citrate de sodium, du sarcosyle et du mercaptoéthanol.

8. Méthode suivant la revendication 6, caractérisée en ce que cette étape d'extraction est conduite avec un mélange de phénol, de chloroforme et d'alcool isoamylique.

9. Méthode suivant la revendication 6, caractérisée en ce que la collection de cet ADN est effectuée par séparation des phases par centrifugation, addition d'un volume égal d'isopropanol à la phase aqueuse, refroidissement à -20°C et centrifugation pour sédimenter l'ADN.

10. Méthode suivant la revendication 6, caractérisée en ce que l'ADN est obtenu par les étapes de lavage du culot avec de l'éthanol froid, séchage du culot d'ADN et de dissolution du culot séché dans une solution de Tris-HCl et d'EDTA.

11. Méthode suivant la revendication 1, caractérisée en ce que l'ADN brut mitochondrial ou génomique est amplifié par synthèse chimique préalable des deux oligonucléotides qui flanquent le segment défini du génome à amplifier; utilisation de ces oligonucléotides comme amorces dans une série de cycles qui comprennent la séparation d'ADN double brin, l'hybridation des amorces sur un ADN servant de matrice et l'élongation du segment défini d'ADN avec une ADN polymérase thermostable et des précurseurs de désoxynucléotide triphosphates; soumission à une électrophorèse du mélange réactionnel, coloration de l'ADN et excision du segment amplifié d'ADN du gel; préparation d'une matrice simple brin par amplification asymétrique; élimination des nucléotides, amorces et sels tampons résiduels de l'ADN simple brin par dialyse centrifuge; et récupération de l'ADN simple brin.

12. Méthode suivant la revendication 10, caractérisée en ce que cette réaction d'amplification est effectuée dans une solution aqueuse contenant du Tris/HCl, du chlorure de magnésium, du 2-mercaptoéthanol, du dATP, du dCTP, du dGTP, du dTTP, chaque amorce et une ADN polymérase procaryote stable à la chaleur.

13. Méthode suivant la revendication 10, caractérisée en ce que cette amplification comprend environ 30 à environ 40 cycles de : environ 45 secondes à environ 92°C comme étape de dénaturation, environ 45 secondes à environ 50°C comme étape d'hybridation et environ 90 secondes à environ 72°C comme étape d'extension.

14. Méthode suivant la revendication 10, caractérisée en ce que cette excision de cet ADN est conduite par électrophorèse préalable du mélange réactionnel dans un gel d'agarose à bas point de fusion fait avec un tampon Tris, acétate de sodium; visualisation de l'ADN par coloration avec du bromure d'éthidium et excision du segment amplifié d'ADN à partir du gel et fusion dans l'eau.

15. Méthode suivant la revendication 10, caractérisée en ce que la matrice simple brin est préparée par amplification asymétrique dans une solution aqueuse contenant du Tris/HCl, du chlorure de magnésium, du 2-mercaptoéthanol, du dATP, du dCTP, du dGTP, du dTTP, chaque amorce et une ADN polymérase procaryote thermostable.

16. Méthode suivant la revendication 1, caractérisée en ce que le séquençage d'ADN du produit amplifié simple brin est effectué avec la procédure de séquençage d'ADN à terminaison de chaîne didésoxy.

17. Méthode suivant la revendication 15, caractérisée en ce que l'amorce limitante utilisée dans la réaction d'amplification simple brin asymétrique, est utilisée comme amorce de séquençage.

18. Méthode suivant la revendication 15, caractérisée en ce que l'analyse couvre en routine au moins 299 nucléotides du segment défini.

19. Séquence complémentaire de la partie du gène cytochrome-b de THUNNUS TUNA ayant la séquence suivante :

*       * **
AGCCAACGAA GGCGGTCATC ATAACTAGGA GTAGGAGTAC 40
*       *       *
TACTCCGATG TTTCATGTTT CTTTGTATAG GTAAGAGCCG 80
*       *       *       *
TAGTAAAGTC CTCGGCCGAT GTGGAAGTAG ATRCAGATAA 120
*       *   *
AGAAGAAAGA GGCCCCGTTT GCGTGGAGGT TCCGGATGAG 160
*   *       *
TCAACCGAAG TTGACATCTC GGCAAATGTG GGCTACTGAG 200
*       *
GCGAAGGCTG ATTCGACATC AGGGGTGTAG TGTATTGCGA 240
*   *
GGAATAGTCC TGTAAGGATT TGAGAAATAA GGCAAAGGCC 280
AAGTAGTGAG CCAAAGTTT 299.

20. Séquence complémentaire suivant la revendication 18 comprenant la partie du gène cytochrome-b de BLUE FIN TUNA ayant la séquence suivante :

AGCCAACGAA GGCGGTCATC ATAACTAGGA GTAGGAGTAC 40
TACTCCGATG TTTCATGTTT CTTTGTATAG GTAAGAGCCG 80
*
TAGTAAAGTC CTCGGCCGAT GTGGAAGTAG ATACAGATAA 120
*
AGAAGAAAGA GGCCCCGTTT GCGTGGAGGT TCCGGATAAG 160
TCAACCGAAG TTGACATCTC GGCAAATGTG GGCTACTGAG 200
GCGAAGGCTG ATTCGACATC AGGGGTGTAG TGTATTGCGA 240
*
GGAATAGTCC TGTAAGGATC TGAGAAATAA GGCAAAGGCC 280
AAGTAGTGAG CCAAAGTTT 299.

21. Séquence complémentaire suivant la revendication 18 comprenant la partie du gène cytochrome-b de YELLOW FIN TUNA ayant la séquence suivante :

AGCCAACGAA GGCGGTCATC ATAACTAGGA GTAGGAGTAC 40
*
TACTCCGATG TTTCATGTTT CCTTGTATAG GTAAGAGCCG 80
*
TAGTAAAGTC CTCGGCCGAT GTGGAAGTAG ATGCAGATAA 120
*
AGAAGAAAGA GGCCCCGTTT GCGTGGAGGT TCCGGATAAG 160
TCAACCGAAG TTGACATCTC GGCAAATGTG GGCTACTGAG 200
GCGAAGGCTG ATTCGACATC AGGGGTGTAG TGTATTGCGA 240
GGAATAGTCC TGTAAGGATT TGAGAAATAA GGCAAAGGCC 280
AAGTAGTGAG CCAAAGTTT 299.

22. Séquence complémentaire suivant la revendication 18 comprenant la partie du gène cytochrome-b de BIG EYE TUNA ayant la séquence suivante :

*
AGCCAACGAA GGCGGTCATC ATAACTAGGA GTAGTAGTAC 40
TACTCCGATG TTTCATGTTT CTTTGTATAG GTAAGAGCCG 80
*
TAGTAAAGTC CTCGGCCGAT GTGGAAGTAG ATACAGATAA 120
AGAAGAAAGA GGCCCCGTTT GCGTGGAGGT TCCGGATGAG 160
TCAACCGAAG TTGACATCTC GGCAAATGTG GGCTACTGAG 200
*
GCGAAGGCTG ATTCGACATC AGGGGTGTAG TGCATTGCGA 240
GGAATAGTCC TGTAAGGATT TGAGAAATAA GGCAAAGGCC 280
AAGTAGTGAG CCAAAGTTT 299.

23. Séquence complémentaire suivant la revendication 18 comprenant la partie du gène cytochrome-b de ALBACORE TUNA ayant la séquence suivante :

*
AGCCAACGAA GGCGGTCATC ATAACTAGAA GTAGGAGTAC 40
*
TACTCCGATG TTTCATGTTT CTTTGTAGAG GTAAGAGCCG 80
* *
TAGTAAAGGC CTCGGCCGAT GTGGAAGTAG ATGCAGATAA 120
AGAAGAAAGA GGCCCCGTTT GCGTGGAGGT TCCGGATGAG 160
TCAACCGAAG TTGACATCTC GGCAAATGTG GGCTACTGAG 200
*
GCGAAGGCTG ATTCGACATC AGGGGTATAG TGTATTGCGA 240
GGAATAGTCC TGTAAGGATT TGAGAAATAA GGCAAAGGCC 280
AAGTAGTGAG CCAAAGTTT 299.

24. Kit comprenant un support, des compartiments à l'intérieur de ce support pour recevoir un récipient contenant de l'ADN provenant d'un organisme eucaryote ou procaryote défini, un deuxième récipient contenant un segment amplifié de l'ADN provenant de cet organisme défini, et un troisième récipient contenant des séquences d'ADN pour au moins l'un de plusieurs genres, espèces, souches, sous-espèces, sous-ensembles et d'ADN en dérivant.

25. Kit suivant la revendication 24, dans lequel l'organisme est un mammifère, un oiseau, un reptile, un amphibien, un poisson ou un invertébré.

26. Kit suivant la revendication 24 comprenant un support, des compartiments à l'intérieur de ce support pour recevoir un récipient contenant de l'ADN provenant d'un organisme défini, un deuxième récipient contenant un segment amplifié de l'ADN provenant de cet organisme défini, et un troisième récipient contenant des séquences d'ADN pour un ensemble de plusieurs espèces ou d'ADN en dérivant.
